(19) Europäisches Patentamt
European Patent Office
Office européen des brevets

(11) Veröffentlichungsnummer : **0 368 063 B1**

(12) # EUROPÄISCHE PATENTSCHRIFT

(45) Veröffentlichungstag der Patentschrift : **10.06.92 Patentblatt 92/24**

(51) Int. Cl.$^5$ : **C07C 17/12**

(21) Anmeldenummer : **89119556.2**

(22) Anmeldetag : **21.10.89**

(54) **Verfahren zur Kernchlorierung von aromatischen Kohlenwasserstoffen.**

(30) Priorität : **05.11.88 DE 3837574**
**05.11.88 DE 3837575**

(43) Veröffentlichungstag der Anmeldung : **16.05.90 Patentblatt 90/20**

(45) Bekanntmachung des Hinweises auf die Patenterteilung : **10.06.92 Patentblatt 92/24**

(84) Benannte Vertragsstaaten : **BE DE FR GB IT**

(56) Entgegenhaltungen :
**EP-A- 0 126 669**
**EP-A- 0 292 824**

(73) Patentinhaber : **BAYER AG**
**W-5090 Leverkusen 1 Bayerwerk (DE)**

(72) Erfinder : **Mais, Franz-Josef, Dr.**
**Gustav-Poensgen-Strasse 23**
**W-4000 Düsseldorf 1 (DE)**
Erfinder : **Fiege, Helmut, Dr.**
**Walter-Flex-Strasse 23**
**W-5090 Leverkusen 1 (DE)**

EP 0 368 063 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Kernchlorierung von aromatischen Kohlenwasserstoffen in Gegenwart von Friedel-Crafts-Katalysatoren und in Gegenwart von Co-Katalysatoren in flüssiger Phase.

Die Umsetzung von aromatischen Kohlenwasserstoffen, wie Toluol, in flüssiger Phase mit gasförmigem Chlor zu kernsubstituierten Chlorderivaten, wie Monochlortoluol, ist bekannt (Ullmanns Enzyklopädie der technischen Chemie, 4. Auflage, Band 9, Seite 499f.). Man führt diese Chlorierung im allgemeinen in Gegenwart von Friedel-Crafts-Katalysatoren, wie Eisen(III)-chlorid, Antimonchloriden oder Aluminiumchlorid, durch. Das erhaltene Chlorierungsprodukt ist gewöhnlich eine Mischung aus isomeren monochlorierten und polychlorierten Verbindungen. Bei Verwendung von $FeCl_3$ erhält man beispielsweise aus Toluol ein Gemisch aus Monochlortoluolen und Dichlortoluolen; in der Monochlortoluolfraktion ist das Hauptprodukt o-Chlortoluol neben p-Chlortoluol und einem geringen Anteil an m-Chlortoluol.

Da besonders p-Chloralkylbenzole, wie p-Chlortoluol, wertvolle Zwischenprodukte darstellen, hat es in der Vergangenheit nicht an Versuchen gefehlt, die Chlorierung so zu lenken, daß das Verhältnis von o- zu p-Chloralkylbenzolen erniedrigt wird, d.h., man versuchte, Bedingungen zu finden, die die Bildung von p-Chloralkylbenzolen begünstigten.

Aus US 3.226.447 ist bekannt, daß durch Zusatz von Schwefelverbindungen mit zweiwertigem Schwefel zum Friedel-Crafts-Katalysator bei der Chlorierung von Toluol ein o/p-Verhältnis von 1,2 erhalten werden kann. Nachteilig bei diesem Verfahren ist die Tatsache, daß man dieses wenig günstige Verhältnis nur bei Anwendung von Antimonsalzen als Friedel-Crafts-Katalysatoren erreicht. Nachteilig ist weiterhin, daß die erforderlichen Mengen der Katalysatorkomponenten gemäß dem dortigen Beispiel 16 sehr hoch liegen, nämlich bei 1 Gew.-% für jeden der beiden katalytischen Zusätze. Wie das o/p-Verhältnis mit einem Wert von >1 zeigt, entsteht hierbei immer noch mehr o- als p-Chlortoluol.

In DE-OS 15 43 020 und US 4.031.144 wird ebenfalls die Chlorierung von Toluol beispielsweise mit $FeCl_3$ und $S_2Cl_2$ beschrieben. Das erhaltene Verhältnis von o/p = 1,03-1,10 ist immer noch unbefriedigend hoch.

In US 4.031.147, US 4.069.263, US 4.069.264 und US 4.250.122 ist die Chlorierung von Toluol mit Friedel-Crafts-Katalysatoren unter Zusatz von Thianthrenen oder substituierten Thianthrenen beschrieben. Die günstigsten erreichbaren o/p-Verhältnisse liegen bei etwa 0,7, werden jedoch entweder nur durch die Verwendung von Antimonsalzen oder im Falle der Verwendung von Eisensalzen nur bei sehr niedrigen Reaktionstemperaturen von etwa 0° C erhalten. Beides ist technisch ausgesprochen ungünstig. So wird die co-katalytische Wirkung der Thianthrene beim Einsatz von Antimonsalzen durch Eisenspuren stark behindert, was in der Technik nur schwer zu realisieren ist. Zudem ist die Reaktion so stark exotherm, daß eine Abführung der Wärme bei etwa 0° C durch Solekühlung sehr aufwendig wird. Ferner werden die Thianthrene unter üblichen Reaktionsbedingungen bereits von allgegenwärtigen Wasserspuren zerstört und verlieren somit ihre Wirksamkeit.

Weiterhin ist aus US 4.289.916, EP 63 384 und EP 173 222 die Chlorierung von Toluol in Gegenwart von Lewis-Säuren und Phenoxathiinen bekannt. Das nach Beispiel 1 von EP 173 222 erreichbare o/p-Verhältnis von 0,6 wird wiederum nur durch die technisch äußerst ungünstige Verwendung von Antimonchlorid und die hohe Menge von 0,29 Gew.-% an Co-Katalysator erreicht. Bei Verwendung von $FeCl_3$ anstelle von Antimonchlorid erhält man ein o/p-Verhältnis von 0,68, allerdings wiederum nur bei der technisch äußerst ungünstigen niedrigen Reaktionstemperatur von 5°C. Bei einer technisch vorteilhaften Reaktionstempe ratur von 50° C steigt das o/p-Verhältnis in Gegenwart von $FeCl_3$ und dem in EP 173 222 beanspruchten Phenoxathiin-Derivat auf 0,88, wie von uns durchgeführte Versuche zeigen (vgl. Beispiel 27). In den genannten US 4.289.916 und EP 63 384 wird ein günstigstes o/p-Verhältnis von etwa 0,8 beschrieben. Auch hier kann das o/p-Verhältnis auf 0,65 gesenkt werden, wenn man anstelle von $FeCl_3$ Antimonchloride und eine Reaktionstemperatur von 20° C, also technisch ungünstige Bedingungen, anwendet. Auch Phenoxathiine werden in Gegenwart von Wasserspuren zerstört.

Aus EP 126 669 ist die Toluol-Chlorierung in Gegenwart von Friedel-Crafts-Katalysatoren und N-substituierten Phenothiazinen bekannt. Das o/p-Verhältnis ist mit 0,84 auch hierbei ungünstig hoch.

Aus EP 112 722, EP 154 236 und EP 248 931 ist die Chlorierung von Toluol in Gegenwart von bestimmten Zeolithen bekannt, wobei unter Zusatz von beispielsweise Halogencarbonsäurehalogeniden als Moderatoren ein o/p-Verhältnis von etwa 0,3 erreicht wird. Nachteilig an diesem Verfahren sind die erheblichen Mengen von 5 Gew.-% Zeolith und 1 Gew.-% an Moderatoren. Wie eigene Versuche zeigten, muß dieses Ergebnis mit dem erheblichen Nachteil erkauft werden, daß in den erhaltenen Gemischen sehr große Mengen (bis zu 8 Gew.-%) an Benzylchloriden auftreten. Die Bildung von Benzylchloriden stört die nachfolgende übliche destillative Aufarbeitung in ganz außerordentlichem Maße.

Es wurde nun ein Verfahren zur Kernchlorierung von aromatischen Kohlenwasserstoffen der Formel

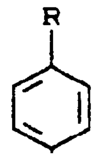

(I),

worin
R geradkettiges oder verzweigtes $C_1$-$C_{12}$-Alkyl oder $C_3$-$C_8$-Cycloalkyl bedeutet,
in Gegenwart von Friedel-Crafts-Katalysatoren und in Gegenwart von Co-Katalysatoren in flüssiger Phase gefunden, das dadurch gekennzeichnet ist, daß man als Co-Katalysatoren cyclische benzokondensierte Imine der Formel

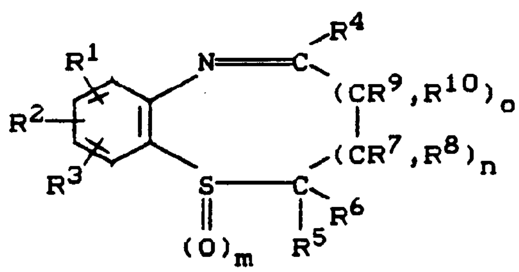

(II),

in der
$R^1$ und $R^2$ unabhängig voneinander Wasserstoff, Hydroxy, Amino, Cyano, Halogen, Nitro, Carboxyl, Halogenocarbonyl, Carboxyamid, Alkoxycarbonyl, Alkyl, Aryl, Alkoxy, Aryloxy, Acyloxy, Alkylthio, Arylthio, Acylthio, Acyl, Thioacyl oder Acylamino bedeuten,
$R^3$ für Wasserstoff oder Chlor steht und weiterhin mit einem der Reste $R^1$ oder $R^2$ bei benachbarter Substitution und gemeinsam mit den substituierten C-Atomen einen anellierten gesättigten, ungesättigten oder aromatischen isocyclischen oder heterocyclischen 5-8-Ring bilden kann,
$R^4$ Wasserstoff, Alkyl, Aryl, Halogen, Alkylthio, Arylthio, Alkoxy, Aryloxy, Amino, Hydrazino, Alkylhydrazino oder Phenylhydrazino bedeutet,
m, n und o unabhängig voneinander den Wert 0 oder 1 annehmen können,
$R^5$, $R^7$ und $R^9$ unabhängig voneinander Wasserstoff, Alkyl, Alkoxy, Phenyl, Acyloxy, Cyano, Halogen, Carboxyl, Alkoxycarbonyl, Phenoxy oder Acyl bedeuten und
$R^6$, $R^8$ und $R^{10}$ unabhängig voneinander Wasserstoff, Alkyl oder Halogen bedeuten, wobei $R^5$ und $R^7$ oder $R^7$ und $R^9$ gemeinsam mit den substituierten C-Atomen einen gesättigten, ungesättigten oder aromatischen isocyclischen oder heterocyclischen 5-8-Ring darstellen können und wobei weiterhin $R^6$ und $R^8$ oder $R^8$ und $R^{10}$ gemeinsam eine Doppelbindung bilden können und wobei weiterhin $R^5$ und $R^6$ gemeinsam doppelt gebundenen Sauerstoff, Schwefel oder $R^{11}$-substituierten Stickstoff darstellen können, wobei $R^{11}$ Alkyl, Aryl, Acyl, Alkylamino oder Arylamino bedeutet,
oder Benzo[f]-1,4-thiazepine der Formel

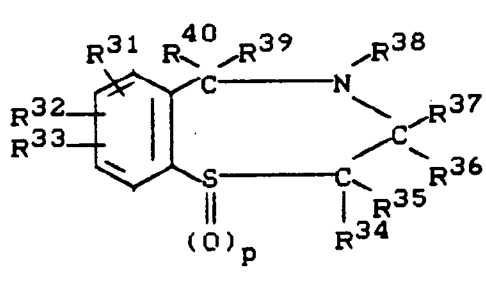

(III),

in der
$R^{31}$ und $R^{32}$ unabhängig voneinander Wasserstoff, Hydroxy, Amino, Cyano, Halogen, Nitro, $C_1$-$C_8$-Alkyl, nicht substituiertes oder durch $R^{31}$ und $R^{32}$ substituiertes Phenyl (mit Ausnahme der erneuten Substitution durch $R^{31}$- und $R^{32}$- substituiertes Phenyl), $C_1$-$C_8$-Alkoxy, Phenoxy, $C_1$-$C_8$-Acyloxy, $C_1$-$C_8$-Acyl oder $C_1$-$C_8$-Alkoxycarbonyl bedeuten,

$R^{33}$ für Wasserstoff oder Chlor steht und weiterhin mit einem der Reste $R^{31}$ oder $R^{32}$ und gemeinsam mit den substituierten C-Atomen einen anellierten gesättigten, ungesättigten oder aromatischen isocyclischen oder heterocyclischen 5-8-Ring bilden kann,
$R^{34}$, $R^{36}$ und $R^{40}$ unabhängig voneinander Wasserstoff, $C_1$-$C_8$-Alkyl, nicht substituiertes oder durch $R^{31}$ und $R^{32}$ substituiertes Phenyl (mit Ausnahme der erneutes Substitution durch $R^{31}$- und $R^{32}$-substituiertes Phenyl), $C_1$-$C_8$-Acyl, $C_1$-$C_8$-Alkoxycarbonyl, Cyano, Halogen, Carboxyl, $C_1$-$C_8$-Alkoxy, $C_1$-$C_8$-Alkylthio, Phenylthio, Benzylthio, Phenoxy oder $C_1$-$C_8$-Acyloxy bedeuten,
$R^{35}$, $R^{37}$ und $R^{39}$ unabhängig voneinander Wasserstoff, $C_1$-$C_8$-Alkyl, Halogen, $C_1$-$C_8$-Alkoxy oder $C_1$-$C_8$-Alkylthio bedeuten,
$R^{38}$ Wasserstoff, $C_1$-$C_8$-Alkyl, nicht substituiertes oder durch $R^{31}$- und $R^{32}$-substituiertes Phenyl (mit Ausnahme der erneuten Substitution durch $R^{31}$- und $R^{32}$-substituiertes Phenyl), $C_1$-$C_8$-Acyl, $C_1$-$C_8$-Thioacyl, Halogencarbonyl oder $C_1$-$C_8$-Alkoxycarbonyl bedeutet und
p für die Zahl Null oder Eins steht,
wobei weiterhin
die Substituentenpaare $R^{34}$ und $R^{35}$, $R^{36}$ und $R^{37}$ sowie $R^{39}$ und $R^{40}$ unabhängig voneinander doppelt gebundenen Sauerstoff, Schwefel oder $R^{38}$-substituierten Stickstoff bedeuten können und wobei weiterhin
die Substituentenpaare $R^{35}$ und $R^{36}$ sowie $R^{38}$ und $R^{39}$ unabhängig voneinander eine Doppelbindung bilden können und wobei weiterhin
die Substituentenpaare $R^{34}$ und $R^{37}$ sowie $R^{38}$ und $R^{39}$ unabhängig voneinander 3- bis 5-gliedriges Alkylen bilden können, bei dem 1 oder 2 C-Atome durch Sauerstoff, Schwefel oder $R^{38}$-substituierten Stickstoff ersetzt sein können, und wobei weiterhin
$R^{40}$ auch die Bedeutung Hydrazino, $C_1$-$C_8$-Alkyl-hydrazino oder Phenyl-hydrazino annehmen kann,
einsetzt.

Die erfindungsgemäß einsetzbaren Co-Katalysatoren der Formeln (II) bzw. (III) besitzen das gemeinsame Merkmal, daß sie benzokondensierte Stickstoff-Schwefel-Heterocyclen sind.

Als Halogen sei Fluor, Chlor, Brom oder Iod, bevorzugt Fluor, Chlor oder Brom, besonders bevorzugt Fluor oder Chlor, genannt.

Als Alkylreste in den genannten Substituenten seien offenkettige mit 1-16 C-Atomen, bevorzugt mit 1-8 C-Atomen, besonders bevorzugt mit 1-4 C-Atomen, und cyclische mit 5-8 C-Atomen, bevorzugt mit 5 oder 6 C-Atomen, genannt. Diese Alkylreste können ihrerseits mit $C_1$-$C_4$-Alkyl, bevorzugt mit Methyl oder Ethyl, substituiert sein, so daß man auch in die Reihe der verzweigten Alkylreste gelangt. Diese Alkylreste können weiterhin durch Fluor, Chlor oder Brom ein- oder mehrfach substituiert sein. Diese Alkylreste können weiterhin durch $C_1$-$C_4$-Alkoxy, bevorzugt mit Methoxy oder Ethoxy, substituiert sein, so daß man in die Reihe der Ether gelangt. Diese Alkylreste können weiterhin durch Phenyl, Naphthyl oder Biphenyl substituiert sein, so daß man in die Reihe der Aralkylreste gelangt. Beispiele für solche Alkylreste sind: Methyl, Ethyl, Propyl, Isopropyl, Butyl, Isobutyl, tert.-Butyl, Amyl, Hexyl, Octyl, Decyl, Dodecyl, Hexadecyl, Cyclopentyl, Methylcyclopentyl, Cyclohexyl, Methylcyclohexyl, Cycloheptyl, Cyclooctyl, Methoxymethyl, Ethoxymethyl, Benzyl, Phenyl-ethyl, Chlormethyl, Dichlormethyl, Trichlormethyl, Trifluormethyl; besonders wichtige Reste sind beispielsweise Methyl, Ethyl, n-Propyl, Benzyl, Trifluormethyl.

Der genannte Bedeutungsumfang für Alkylreste gilt grundsätzlich auch für Alkoxy und Alkylthio sowie in entsprechender Weise für Acyl und Acyloxy; bevorzugt sind Reste mit 1-6 C-Atomen, besonders bevorzugt solche mit 1-4 C-Atomen, wie Methoxy, Ethoxy, tert.-Butoxy, Cyclohexyloxy, Trifluormethoxy, Methylthio, Ethylthio, Cyclohexylthio, Trifluromethylthio, Trichlormethylthio, Formyl, Acetyl, Propionyl, Acetyloxy, Propionyloxy, Trichloracetyl, Trifluoracetyl, Benzoyl, Chlorbenzoyl, Chloracetyl.

Als Arylreste in den obigen Substituenten seien beispielsweise Phenyl, Naphthyl oder Biphenyl genannt, die ihrerseits durch Fluor, Chlor, Brom, Nitro, $C_1$-$C_4$-Alkyl oder $C_1$-$C_4$-Alkoxy substituiert sein können, beispielsweise Phenyl, Naphthyl, Tolyl, Anisyl, Chlorphenyl, Nitrophenyl; besonders wichtig sind beispielsweise Phenyl und Chlorphenyl.

Bezüglich der Aryloxy- und Arylthioreste gilt analog das oben zu den Alkoxy-und Alkylthioresten Gesagte.

Für den Fall, daß $R^3$ mit einem der Reste $R^1$ oder $R^2$ und gemeinsam mit den substituierten C-Atomen einen Ring bildet, kann dieser isocyclisch und gesättigt, ungesättigt oder aromatisch sein oder auch durch einen Gehalt an N-, O- und/oder S-Atomen heterocyclisch sein.

Solche Ringe habe 5-8, bevorzugt 5 oder 6 Ringglieder und sind an den in Formel (III) gezeigten Benzolkern anelliert. Als Beispiele seien genannt: Benzo, Naphthalino, Thieno, Furano, Pyrrolo, Pyridino, Cyclohexano, Cyclopentano, Oxolano, Dioxolano, bevorzugt Benzo und Cyclohexano.

Für den Fall, daß $R^{33}$ mit einem der Reste $R^{31}$ oder $R^{32}$ und gemeinsam mit den substituierten C-Atomen einen Ring bildet, kann dieser isocyclisch und gesättigt, ungesättigt oder aromatisch sein oder auch durch einen Gehalt an N-, O- und/oder S-Atomen heterocyclisch sein. Solche Ringe haben 5-8, bevorzugt 5 oder 6 Ringglieder und sind an den in Formel (III) gezeigten Benzolkern anelliert. Als Beispiele seien genannt: Benzo, Naphthalino, Thieno, Furano, Pyrrolo, Pyridino, Cyclohexano, Cyclopentano, Oxolano, Dioxolano, bevorzugt Benzo und Cyclohexano.

$C_1$-$C_8$-Thioacyl leitet sich von $C_1$-$C_8$-Acyl durch Ersatz des Sauerstoffs durch Schwefel ab.

Halogencarbonyl kann Fluorcarbonyl, Chlorcarbonyl oder Bromcarbonyl, bevorzugt Chlorcarbonyl, sein.

Die im einzelnen angegebenen Substituentenpaare können unabhängig voneinander doppelt gebundenen Sauerstoff, Schwefel oder $R^{38}$-substituierten Stickstoff bedeuten, die näher bezeichneten Doppelbindungen oder 3- bis 5-gliedriges Alkylen bilden, wobei im letzteren Falle anellierte cycloaliphatische Ringe entstehen können, die im Falle der Anellierung an der 2- und 3-Position des Skeletts nach Formel (III) auch eine olefinische Bindung enthalten können. Ferner können solche anellierten Ringe nicht-aromatische heterocyclische Ringe sein, wenn in der Alkylenkette 1 oder 2 C-Atome durch Sauerstoff, Schwefel oder $R^{38}$-substituierten Stickstoff ersetzt sind.

Die als Co-Katalysatoren erfindungsgemäß einzusetzenden cyclischen benzokondensierten Imine der Formel (II) sind gekennzeichnet durch das mit dem N-Atom doppelt verbundene und durch $R^4$ substituierte C-Atom. Zwischen dieses soeben besprochene C-Atom und das dem S-Atom benachbarte C-Atom kann eine einfache Bindung oder 1 oder 2 weitere C-Atome im Sinne der Definition des Index o und n treten. Damit fallen unter die Formel (II) cyclische benzokondensierte Imine, deren Ringsystem 6-, 7- oder 8-gliedrig sein kann und mit folgenden Formeln darstellbar ist:

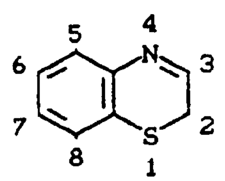
,
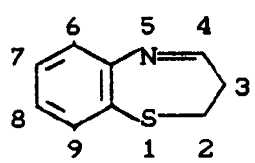
,
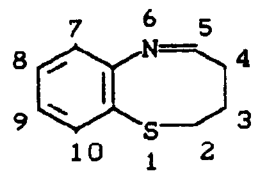
,

(IIa) (IIb) (IIc)

In den Formeln (IIa), (IIb) und (IIc) ist die Bezifferung der das Gerüst bildenden Atome angegeben, während zur besseren Übersichtlichkeit die möglichen Substituenten fortgelassen wurden.
Eine keineswegs erschöpfende Aufzählung für solche erfindungsgemäß einsetzbaren Co-Katalysatoren ist die folgende:
3-Methylthio-2H-1,4-benzothiazin,
3-Benzylthio-2H-1,4-benzothiazin,
3-Methoxy-2H-1,4-benzothiazin,
2-Methyl-3-methylthio-2H-1,4-benzothiazin,
3-Ethylthio-2H-1,4-benzothiazin-2-on,
3-Acetylthio-2H-1,4-benzothiazin,
2-Phenyl-3-methylthio-2H-1,4-benzothiazin,
3-Methylthio-6-methyl-2H-1,4-benzothiazin,
3-Methylthio-6-chlor-2H-1,4-benzothiazin,
3-Methylthio-7-chlor-2H-1,4-benzothiazin,
5,7-Dichlor-3-ethylthio-2H-1,4-benzothiazin,
6,8-Dimethyl-3-methylthio-2H-1,4-benzothiazin,
7-Trifluormethyl-3-methoxy-2H-1,4-benzothiazin,
7-Trifluormethyl-3-methylthio-2H-1,4-benzothiazin,
5,7-Dichlor-2,6-dimethyl-3-methylthio-2H-1,4-benzothiazin
3-Ethoxy-2H-1,4-(2,3-naphthaleno)thiazin,
3,6-Dimethoxy-2H-1,4-benzothiazin,
6-Methoxy-3-propylthio-2H-1,4-benzothiazin,
3-Methylthio-6-methoxy-2H-1,4-benzothiazin,
3-Methylthio-1-oxo-2H-1,4-benzothiazin,
4-Methylthio-2,3-dihydro-1,5-benzothiazepin,
6,8-Dichlor-4-methylthio-2,3-dihydro-1,5-benzothiazepin,
7-Trifluormethyl-4-methylthio-2,3-dihydro-1,5-benzothiazepin,
8-Methoxy-4-methylthio-2,3-dihydro-1,5-benzothiazepin,
7-Methoxy-4-methylthio-2,3-dihydro-1,5-benzothiazepin,
4-Methoxy-2,3-dihydro-1,5-benzothiazepin,

4-Methylthio-1,5-benzothiazepin-2(3H)-on,
4-Ethylthio-2,3-dihydro-1,5-benzothiazepin,
4-Methyl-2,3-dihydro-1,5-benzothiazepin
2,2,4-Trimethyl-2,3-dihydro-1,5-benzothiazepin,
4-Methyl-2,3-tetramethylen-1,5-benzothiazepin,
4-Phenylhydrazino-2,3-dihydro-1,5-benzothiazepin,
4-Methylthio-7,9-dimethyl-2,3-dihydro-1,5-benzothiazepin,
4-Benzylthio-2,3-dihydro-1,5-benzothiazepin,
4-Methylthio-7,8-dimethyl-2,3-dihydro-1,5-benzothiazepin,
2-Methyl-4-methylthio-2,3-dihydro-1,5-benzothiazepin,
2,3-Dimethyl-4-methylthio-2,3-dihydro-1,5-benzothiazepin,
2,3-Tetramethylen-4-methylthio-2,3-dihydro-1,5-benzothiazepin,
2,3-Trimethylen-4-methylthio-2,3-dihydro-1,5-benzothiazepin,
7-Methoxy-4-methylthio-2,3-tetramethylen-2,3-dihydro-1,5-benzothiazepin,
7,8-Dimethyl-4-methylthio-2,3-tetramethylen-2,3-dihydro-1,5-benzothiazepin,
7,9-Dimethyl-4-methylthio-2,3-tetramethylen-2,3-dihydro-1,5-benzothiazepin,
2,3-Tetramethylen-4-methoxy-2,3-dihydro-1,5-benzothiazepin,
7,9-Dimethyl-4-methoxy-2,3-tetramethylen-2,3-dihydro-1,5-benzothiazepin,
6,8-Dichlor-7-methyl-4-methylthio-2,3-dihydro-1,5-benzothiazepin,
7-Chlor-4-methoxy-2,3-dihydro-1,5-benzothiazepin,
4-Methylthio-1-oxo-2,3-dihydro-1,5-benzothiazepin,
2-Phenyl-4-methylthio-2,3-dihydro-1,5-benzothiazepin,
4,7,8-Trimethoxy-2-phenyl-2,3-dihydro-1,5-benzothiazepin,
8,9-Dichlor-7-methyl-4-methoxy-2,3-trimethylen-2,3-dihydro-1,5-benzothiazepin,
2-Methyl-4-methylthio-1,5-benzothiazepin,
4-Methylthio-1,5-benzothiazepin,
4-Methylthio-1,5-dibenzo-[b,f]thiazepin,
2,3-Dichlor-4-methylthio-1,5-benzothiazepin,
7,8-Dimethyl-2-phenyl-4-methoxy-2,
3-dihydro-1,5-benzothiazepin,
3-Acetoxy-4-methylthio-2,3-dihydro-1,5-benzothiazepin,
3-Acetamido-4-methylthio-2,3-dihydro-1,5-benzothiazepin,
2-Propyl-4-methylthio-2,3-dihydro-1,5-benzothiazepin,
2-Propyl-4-propylthio-2,3-dihydro-1,5-benzothiazepin,
4-Methylthio-1,5-(1,2-naphthaleno)thiazepin,
4-Methoxy-1,5-benzothiazepin,
4-Acetylthio-2,3-dihydro-1,5-benzothiazepin,
2,3-Dimethyl-4-acetyl-2,3-dihydro-1,5-benzothiazepin,
4-Propionylthio-2,3-dihydro-1,5-benzothiazepin,
4-Propionyloxy-2,3-dihydro-1,5-benzothiazepin,
4-Chlorcarbonylthio-2,3-dihydro-1,5-benzothiazepin,
4-Benzoylthio-2,3-dihydro-1,5-benzothiazepin,
7,9-Dimethyl-4-acetyloxy-2,3-dihydro-1,5-benzothiazepin,
5-Methylthio-3,4-dihydro-2H-1,6-benzothiazocin,
2-Methyl-5-methylthio-3,4-dihydro-2H-1,6-benzothiazocin,
2-Phenyl-5-methylthio-3,4-dihydro-2H-1,6-benzothiazocin,
5-Methoxy-2H-3,4-dihydro-1,6-benzothiazocin,
8,10-Dimethyl-5-methylthio-2H-3,4-dihydro-1,6-benzothiazocin,
5-Methylthio-2,3-tetramethylen-2H-3,4-dihydro-1,6-benzothiazocin,
2,3-Dimethyl-5-methylthio-2H-3,4-dihydro-1,6-benzothiazocin,
5-Ethylthio-2H-3,4-dihydro-1,6-benzothiazocin,
5-Methylthio-2H-3,4-dihydro-1,6-benzothiazocin-2-on,
8-Methoxy-5-methylthio-2H-3,4-dihydro-1,6-benzothiazocin,
5-Methylthio-2H-1,6-dibenzo[c,g]thiazocin,
5-Methylthio-3,4-tetramethylen-2H-3,4-dihydro-1,6-benzothiazocin,
5-Methylthio-1-oxo-2H-3,4-dihydro-1,6-benzothiazocin,
7,9-Dichlor-5-ethoxy-2H-3,4-dihydro-1,6-benzothiazocin,
2-Chlor-5-methylthio-4H-1,6-benzothiazocin,

5-Methylthio-2H-3,4-dihydro-1,6-benzothiazocin-2-thion,
5-Methylthio-4H-1,6-dibenzo[b,g]thiazocin,
5-Methoxy-8-trifluormethyl-2H-3,4-dihydro-1,6-benzothiazocin,
4-Methyl-5-ethylthio-2H-3,4-dihydro-1,6-benzothiazocin,
5-Methylthio-2H-1,6-benzothiazocin-2-on,
5-Methylthio-2H-3,4-dihydro-1,6(1,2-naphthaleno)thiazocin,

bevorzugt:

4-Methylthio-2,3-dihydro-1,5-benzothiazepin,
6,8-Dichlor-4-methylthio-2,3-dihydro-1,5-benzothiazepin,
7-Trifluormethyl-4-methylthio-2,3-dihydro-1,5-benzothiazepin,
8-Methoxy-4-methylthio-2,3-dihydro-1,5-benzothiazepin,
7-Methoxy-4-methylthio-2,3-dihydro-1,5-benzothiazepin,
4-Methylthio-7,9-dimethyl-2,3-dihydro-1,5-benzothiazepin,
4-Methylthio-7,8-dimethyl-2,3-dihydro-1,5-benzothiazepin,
2-Methyl-4-methylthio-2,3-dihydro-1,5-benzothiazepin,
2,3-Dimethyl-4-methylthio-2,3-dihydro-1,5-benzothiazepin,
2,3-Tetramethylen-4-methylthio-2,3-dihydro-1,5-benzothiazepin,
2,3-Trimethylen-4-methylthio-2,3-dihydro-1,5-benzothiazepin,
7-Methoxy-4-methylthio-2,3-tetramethylen-2,3-dihydro-1,5-benzothiazepin,
7,8-Dimethyl-4-methylthio-2,3-tetramethylen-2,3-dihydro-1,5-benzothiazepin,
4-Methyl-2,3-dihydro-1,5-benzothiazepin,
4-Methyl-2,3-tetramethylen-2,3-dihydro-1,5-benzothiazepin,
4-Phenylhydrazino-2,3-dihydro-1,5-benzothiazepin
7,9-Dimethyl-4-methylthio-2,3-tetramethylen-2,3-dihydro-1,5-benzothiazepin,
7,9-Dimethyl-4-methoxy-2,3-tetramethylen-2,3-dihydro-1,5-benzothiazepin,
8,9-Dichlor-7-methyl-4-methoxy-2,3-trimethylen-2,3-dihydro-1,5-benzothiazepin,
2-Propyl-4-methylthio-2,3-dihydro-1,5-benzothiazepin,
4-Acetylthio-2,3-dihydro-1,5-benzothiazepin.

Bevorzugte erfindungsgemäß einsetzbare cyclischen benzokondensierte Imine sind solche der Formeln

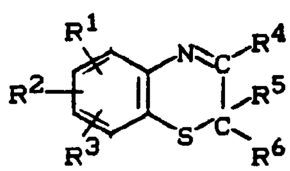

(IV),

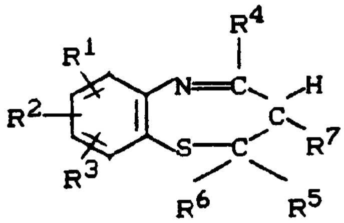

(V)

und/oder

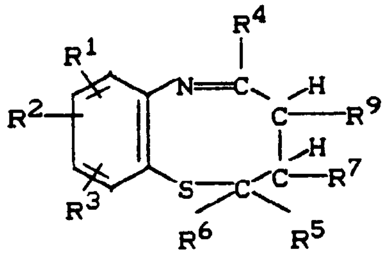

(VI)

In den Formeln (IV), (V) und (VI) dieser bevorzugten Co-Katalysatoren haben $R^1$ bis $R^9$ den obengenannten Bedeutungsumfang.

Besonders bevorzugte Co-Katalysatoren sind solche der Formeln (IV), (V) und (VI), in denen anstelle von $R^1$, $R^2$ und $R^3$ die Reste $R^{12}$, $R^{13}$ und $R^{14}$ treten, in denen
$R^{12}$ und $R^{13}$ unabhängig voneinander Wasserstoff, Halogen, Nitro, Alkyl, Aryl, Alkoxy, Aryloxy, Acyloxy, Alkylthio, Arylthio oder Acyl bedeuten und
$R^{14}$ für Wasserstoff oder Chlor steht und weiterhin mit einem der Reste $R^{12}$ oder $R^{13}$ und gemeinsam mit den durch sie substituierten C-Atomen einen anellierten, gesättigten isocyclischen 5-7-Ring oder einen anellierten Benzolring bilden kann.

In ganz besonders bevorzugter Weise werden Co-Katalysatoren der Formeln (IV), (V) und (VI) eingesetzt, in denen anstelle von $R^{12}$, $R^{13}$ und $R^{14}$ die Reste $R^{21}$, $R^{22}$ und $R^{23}$ treten, von denen $R^{21}$ und $R^{22}$ unabhängig voneinander Wasserstoff, $C_1$-$C_4$-Alkyl, $C_1$-$C_4$-Alkoxy, Fluor oder Chlor bedeuten und
$R^{23}$ für Wasserstoff oder Chlor steht und weiterhin mit einem der Reste $R^{21}$ oder $R^{22}$ und gemeinsam mit dem durch sie substituierten C-Atomen einen anellierten Benzolring bilden kann.

Weitere erfindungsgemäß einsetzbare Co-Katalysatoren sind solche der Formel (IV), (V) und (VI), in denen an die Stelle von $R^4$ der Rest $R^{15}$ tritt, der Wasserstoff, $C_1$-$C_4$-Alkyl, $C_1$-$C_4$-Alkoxy, Phenyl, $C_1$-$C_4$-Acyl, $C_1$-$C_4$-Alkylthio, Benzyl, Benzyloxy, Benzylthio, Hydrazino, $C_1$-$C_4$-Alkylhydrazino, Phenylhydrazino oder Chlor bedeutet.

Besonders bevorzugt sind Co-Katalysatoren der Formeln (IV), (V) und (VI), in denen anstelle von $R^{15}$ der Reste $R^{24}$ tritt, der Wasserstoff, $C_1$-$C_4$-Alkyl, Benzyl, Phenyl, $C_1$-$C_4$-Alkoxy, $C_1$-$C_4$-Alkylthio, Benzyloxy, Benzylthio, Hydrazino oder Phenylhydrazino bedeutet.

Weitere bevorzugte erfindungsgemäß einsetzbare Co-Katalysatoren sind solche der Formeln (IV), (V) und (VI), in denen anstelle von $R^5$, $R^6$, $R^7$ und $R^9$ die Reste $R^{16}$, $R^{17}$, $R^{18}$ und $R^{19}$ treten, die unabhängig voneinander Wasserstoff, $C_1$-$C_4$-Alkyl, Fluor oder Chlor bedeuten, wobei $R^{16}$, $R^{18}$ und $R^{19}$ zusätzlich $C_1$-$C_4$-Alkoxy, $C_1$-$C_4$-Acyl oder Phenyl bedeuten können und weiterhin bei benachbarter Substitution gemeinsam mit den substituierten C-Atomen einen Cyclopentan-, Cyclohexan-oder Benzolring bilden können.

In besonders bevorzugter Weise werden cyclische benzokondensierte Imine der Formel (V) eingesetzt.

Die erfindungsgemäß einzusetzenden cyclischen benzokondensierten Imine können nach an sich bekannten Verfahren hergestellt werden. Beispielsweise können cyclische Thioamide metalliert und mit elektrophilen Reagenzien zu den Iminen umgesetzt werden (Chem. Ber. 102, 1869 (1969), J. Heterocyclic Chem. 20, 1593 (1985). Weiterhin können o-Aminothiophenole mit $\alpha$, $\beta$-ungesättigten Ketenen oder dimeren Ketenen direkt zu cyclischen Iminen umgesetzt werden. (US 3 125 563, Chem. Ber. 90, 2683 (1957), Khim. Geterotsikl. Soedin 1968, 468).

Die anderen einsetzbaren Co-Katalysatoren der Formel (III) sind an der [f]-Bindung des 1,4-Thiazepinsystems benzokondensierte Verbindungen, deren Grundgerüst mit Bezeichnung der Atompositionen und Bindungen das folgende ist:

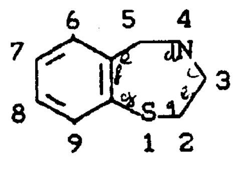

Als Illustration für solche Benzo[f]-1,4-thiazepine dient die folgende, keineswegs erschöpfende Aufzählung:
4-Acetyl-2,3-dihydro-1,4-benzothiazepin-5(4H)-on
4-Chlorcarbonyl-2,3-dihydro-1,4-benzothiazepin-5(4H)-on

5-Methoxy-2,3-dihydro-1,4-benzothiazepin
2,3-Dimethyl-2,3-dihydro-5-methoxy-1,4-benzothiazepin
2,3,5-Trimethyl-2,3-dihydro-1,4-benzothiazepin
2,3-Dihydro-1,4-benzothiazepin-5(4H)-on-1-oxid
2,3-Dihydro-1,4-benzothiazepin-5(4H)-thion
2,3-Dihydro-1,4-benzothiazepin-5(4H)-on
4-Methyl-2,3-dihydro-1,4-benzothiazepin-5(4H)-on
2,3-Tetramethylen-2,3-dihydro-1,4-benzothiazepin-5(4H)-on
2,3-Tetramethylen-2,3,4,5-tetrahydro-1,4-benzothiazepin
4-Ethyl-2,3,4,5-tetrahydro-1,4-benzothiazepin
2,4-Dimethyl-2,3,4,5-tetrahydro-1,4-benzothiazepin
7-Methoxy-2,3-tetramethylen-2,3-dihydro-1,4-benzothiazepin-5(4H)-on
7,9-Dimethyl-2,3-tetramethylen-2,3-dihydro-1,4-benzothiazepin-5(4H)-on
2,3-Tetramethylen-4-methylthio-2,3-dihydro-1,4-benzothiazepin
7,9-Dimethyl-2,3-tetramethylen-4-methyl-thio-2,3-dihydro-1,4-benzothiazepin
2-Phenylthio-2,3-dihydro-1,4-benzothiazepin-5(4H)-on
5-Ethylthio-2,3-dihydro-1,4-benzothiazepin
5-Methylthio-2,3-dihydro-1,4-benzothiazepin
5-Benzylthio-2,3-dihydro-1,4-benzothiazepin
5-Methyl-2,3-dihydro-1,4-benzothiazepin
3-Acetyloxy-2,3-dihydro-1,4-benzothiazepin-5(4H)-on
2,3-Dimethyl-7-nitro-2,3-dihydro-1,4-benzothiazepin-5(4H)-on
4-Chlorocarbonyl-2,3,4,5-tetrahydro-1,4-benzothiazepin
1,4-Benzothiazepin-2(3H)-5(4H)-dion
1,4-Benzothiazepin-2(3H)-5(4H)-dithion
4-Acetyl-2,3-tetramethylen-2,3,4,5-tetrahydro-1,4-benzothiazepin
5,7-Dimethoxy-2,3-dihydro-1,4-benzothiazepin-5(4H)-on
7-Methylthio-2,3-dihydro-1,4-benzothiazepin-5(4H)-on
7,8-Dimethylthio-2,3-dihydro-1,4-benzothiazepin-5(4H)-on
7,8-Tetramethylen-2,3-dihydro-1,4-benzothiazepin-5(4H)-on
5-Methyl-2,3-dihydro-1,4-benzothiazepin-1-oxid
2-Methyl-2,3-dihydro-1,4-benzothiazepin-5(4H)-on
2,7,9-Trimethyl-2,3-dihydro-1,4-benzothiazepin-5(4H)-on
2,7,9-Trimethyl-5-methoxy-2,3-dihydro-1,4-benzothiazepin
2,7,9-Trimethyl-5-β-phenylhydrazino-2,3-dihydro-1,4-benzothiazepin
3-Methyl-2,3-dihydro-1,4-benzothiazepin-5(4H)-on
7,9-Dimethyl-8-chlor-2,3-dihydro-1,4-benzothiazepin-5(4H)-on
8-Trifluormethyl-2,3-dihydro-1,4-benzothiazepin-5(4H)-on
7,8-Dimethyl-4-trifluoracetyl-2,3-dihydro-1,4-benzothiazepin-5(4H)-on
2-Acetyl-2,3-dihydro-1,4-benzothiazepin-5(4H)-on
5-β-Phenylhydrazino-2,3-dihydro-1,4-benzothiazepin
2-Chlor-2,3-dihydro-1,4-benzothiazepin-5(4H)-on
2-Chlor-1,4-benzothiazepin-5(4H)-on
3-Methyl-1,4-benzothiazepin-5(4H)-on
3,7,9-Trimethyl-1,4-benzothiazepin-5(4H)-on
7-Methyl-2,3-dihydro-1,4-benzothiazepin-5(4H)-on
7-Methyl-8-chlor-2,3-dihydro-1,4-benzothiazepin-5(4H)-on
4-Acetyl-2,3,4,5-tetrahydro-1,4-benzothiazepin
2,3,4,5-Tetrahydro-1,4-benzothiazepin
2,3-Dimethyl-2,3,4,5-tetrahydro-1,4-benzothiazepin
7,9-Dichlor-2,3-dihydro-1,
4-benzothiazepin-5(4H)-on
7-Nitro-2,3-dihydro-1,4-benzothiazepin-5(4H)-on
8-Cyano-2,3-dihydro-1,4-benzothiazepin-5(4H)-on
8-Chlor-2,3-dihydro-1,4-benzothiazepin-5(4H)-on
7,9-Dibrom-2,3-dihydro-1,4-benzothiazepin-5(4H)-on
3-Acetyl-2,3-dihydro-1,4-benzothiazepin-5(4H)-on
2-Phenyl-3-methyl-2,3-dihydro-1,4-benzothiazepin-5(4H)-on

4-Methoxy-7-methyl-2,3-dihydro-1,4-benzothiazepin

4-β-Phenylhydrazino-7-methyl-2,3-dihydro-1,4-benzothiazepin

Bevorzugte Benzo[f]-1,4-thiazepine sind solche, in denen das Substituentenpaar $R^{39}$ und $R^{40}$ doppelt gebundenen Sauerstoff, Schwefel oder $R^{38}$-substituierten Stickstoff bedeutet und die übrigen Substituenten und Zeichen den oben bezeichneten Umfang haben. Solche Co-Katalysatoren lassen sich durch die Formel

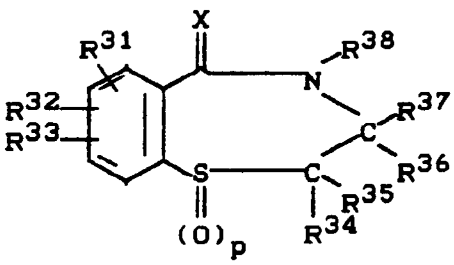

(VII)

darstellen, worin

X doppelt gebundenen Sauerstoff, Schwefel oder $R^{38}$-substituierten Stickstoff bedeutet und $R^{31}$ bis $R^{38}$ und der Index p den oben angegebenen Umfang haben.

In weiterhin bevorzugter Weise werden Co-Katalysatoren eingesetzt, in denen das Substituentenpaar $R^{38}$ und $R^{39}$ eine Doppelbindung darstellt, wobei gleichzeitig der Substituent $R^{40}$ in besonders bevorzugter Weise den Umfang von $R^{41}$ annimmt. Solche Co-Katalysatoren können durch die Formel

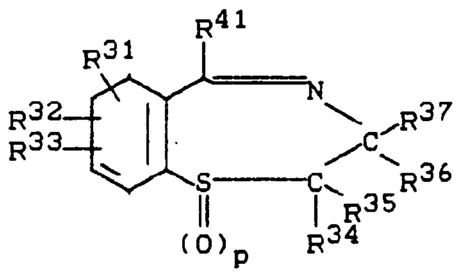

(VIII)

dargestellt werden, in der

$R^{41}$ $C_1$-$C_8$-Alkyl, $C_1$-$C_8$-Alkoxy, $C_1$-$C_8$-Alkylthio, Hydrazino, β-Phenyl-hydrazino oder β-$C_1$-$C_8$-Alkyl-hydrazino bedeutet und

$R^{31}$ bis $R^{37}$ und der Index p den obengenannten Bedeutungsumfang haben.

Weitere bevorzugte Co-Katalysatoren sind solche, in denen das Substituentenpaar $R^{34}$ und $R^{37}$ 3- bis 5-gliedriges Alkylen bedeutet und somit einen anellierten 5- bis 7-gliedrigen Ring bildet. Solche Co-Katalysatoren lassen sich durch die Formel

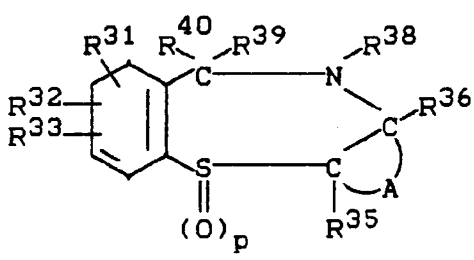

(IX)

darstellen, in der

A Trimethylen, Tetramethylen oder Pentamethylen bedeutet und $R^{31}$, $R^{32}$, $R^{33}$, $R^{35}$, $R^{36}$, $R^{38}$, $R^{39}$, $R^{40}$ und p den obengenannten Bedeutungsumfang haben.

In weiterhin bevorzugter Form werden Co-Katalysatoren eingesetzt, in denen der Index p den Wert Null annimmt. Sie sind durch die Formel

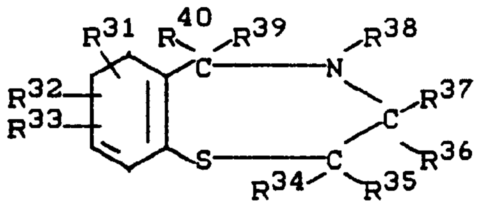

(X)

darstellbar, worin die Reste $R^{31}$ bis $R^{40}$ den obengenannten Bedeutungsumfang annehmen.

In weiterhin bevorzugter Form werden Co-Katalysatoren der Formel (III) eingesetzt, in denen anstelle von $R^{31}$, $R^{32}$ und $R^{33}$ die Reste $R^{42}$, $R^{43}$ bzw. $R^{44}$ treten, von denen

$R^{42}$ und $R^{43}$ unabhängig voneinander Wasserstoff, Halogen, Nitro, Alkyl, Aryl, Alkoxy, Aryloxy, Acyloxy, Alkylthio, Arylthio, Acyl oder Thioacyl bedeuten und

$R^{44}$ für Wasserstoff oder Chlor steht und weiterhin mit einem der Reste $R^{42}$ und $R^{43}$ und gemeinsam mit den substituierten C-Atomen einen anellierten gesättigten isocyclischen 5-7-Ring oder einen anellierten Benzolring bilden kann.

In ganz besonders bevorzugter Weise werden Co-Katalysatoren der Formel (III) eingesetzt, in denen anstelle von $R^{42}$, $R^{43}$ und $R^{44}$ die Reste $R^{51}$, $R^{52}$ bzw. $R^{53}$ treten, von denen

$R^{51}$ und $R^{52}$ unabhängig voneinander Wasserstoff, $C_1$-$C_4$-Alkyl, $C_1$-$C_4$-Alkoxy, Fluor oder Chlor bedeuten und

$R^{53}$ für Wasserstoff oder Chlor steht und weiterhin mit einem der Reste $R^{51}$ oder $R^{52}$ und gemeinsam mit den substituierten C-Atomen einen anellierten Cyclopentan-, Cyclohexan- oder Benzolring bilden kann.

Die erfindungsgemäß als Co-Katalysatoren einzusetzenden Benzo[f]-1,4-thiazepine können nach bekannten Verfahren hergestellt werden, beispielsweise durch Umsetzung von o-Mercaptobenzoesäureestern mit gegebenenfalls substituiertem Ethylenimin oder durch Umsetzung von o-Mercaptobenzoesäuren mit β-Aminoalkoholsulfaten und anschließendem Ringschluß.

Als Beispiele für die erfindungsgemäß im Kern zu chlorierenden aromatischen Kohlenwasserstoffe der Formel (I) seien genannt: Toluol, Ethylbenzol, Propylbenzol, Cumol, tert.-Butylbenzol und Phenylcyclohexan; besonders wichtig ist das Verfahren für die Kernchlorierung von Toluol.

Das erfindungsgemäße Verfahren wird in flüssiger Phase durchgeführt, wobei der aromatische Kohlenwasserstoff in flüssiger (geschmolzener) Form oder gegebenenfalls in Verdünnung mit einem inerten Lösungsmittel eingesetzt werden kann. Geeignete Lösungsmittel sind solche, die durch Chlor unter den Bedingungen einer Kernchlorierung nicht angegriffen werden und dem Fachmann hierfür bekannt sind, wie Methylenchlorid, Chloroform, Tetrachlorkohlenstoff, Essigsäure. In bevorzugter Weise wird ohne Lösungsmittel gearbeitet.

Als Chlorierungsmittel für das erfindungsgemäße Verfahren wird vorzugsweise Chlor verwendet. Das Chlor kann flüssig oder gasförmig in das Reaktionsgemisch eingeleitet werden. Bevorzugt wird gasförmiges Chlor eingesetzt.

Es können jedoch auch andere Chlorierungsmittel verwendet werden, die, wie beispielsweise Sulfurylchlorid, unter den Reaktionsbedingungen Chlor abgeben.

Die erfindungsgemäß durchzuführende Kernchlorierung kann grundsätzlich bei einer Temperatur vom Erstarrungspunkt bis zum Siedepunkt des Reaktionsgemisches durchgeführt werden. Im allgemeinen liegt die Reaktionstemperatur bei 0-100° C, bevorzugt 20-80° C, besonders bevorzugt 40-60° C.

Der Reaktionsdruck kann normal, vermindert oder erhöht sein und ist grundsätzlich unkritisch. Wegen der kostengünstigen Durchführung ist Normaldruck bevorzugt. Erhöhter Druck kann beispielsweise dann angezeigt sein, wenn oberhalb des Siedepunktes eines tiefsiedenden Lösungsmittels gearbeitet werden soll; in diesem Fall kann beispielsweise unter dem sich automatisch einstellenden Eigendruck des Reaktionsgemisches gearbeitet werden. Der Chlorierungsgrad des Reaktionsgemisches liegt bevorzugt nicht höher als 1, bezogen auf den zu chlorierenden aromatischen Kohlenwasserstoff. Höhere Chlorierungsgrade sind möglich, aber gewöhnlich nicht vorteilhaft, da sie zur Bildung unerwünschter mehrfach chlorierter Produkte führen. Chlor bzw. eine chlorabgebende Substanz werden daher beispielsweise in einer Menge von 0,8-1,1, bevorzugt 0,8-1,0 Mol pro Mol des aromatischen Kohlenwasserstoffs eingesetzt.

Friedel-Crafts-Katalysatoren für das erfindungsgemäße Verfahren sind alle bekannten, beispielsweise Antimonchloride, Antimonoxichlorid, Aluminiumchlorid, Eisen(II)-chlorid, Eisen(III)-chlorid, Tellurchloride, Molybdänchloride, Wolframchloride, Titanchloride, Zinkchlorid, Zinnchloride, Borchlorid und/oder Bortrifluorid. Es können jedoch auch Elemente und Elementverbindungen, die während der Chlorierung einen Friedel-Crafts-Katalysator (Lewis-Säure) bilden, eingesetzt werden. Beispielsweise die elementaren Metalle oder Halbmetalle Antimon, Eisen, Blei, Zinn, Zink, Molybdän, Tellur und Aluminium oder deren Oxide, Sulfide, Carbonyle oder Salze (beispielsweise Carbonate oder ähnliche); genannt seien beispielsweise Antimonoxide,

Eisenoxide, Eisensulfide, Bleisulfide, Zinnsulfide, Zinksulfide, Eisencarbonyle, Molybdäncarbonyle und/oder Borphosphat. Anstelle der erwähnten Chloride können auch die Bromide, gegebenenfalls auch die Fluoride oder Iodide der genannten Elemente eingesetzt werden. Bevorzugte Friedel-Crafts-Katalysatoren sind Antimonchloride, Aluminiumchloride, Eisen, Eisenoxide, Eisensulfide, Eisencarbonyle und/oder Eisen(III)-chlorid. Besonders bevorzugt ist Eisen(III)-chlorid.

Die Mengen des Friedel-Crafts-Katalysators oder eines Gemisches mehrerer von ihnen können in weiten Grenzen variiert werden. So ist bereits bei einem Zusatz von 0,0005 Gew.-% eine Katalysatorwirkung erkennbar; andererseits können auch 5 Gew.-% oder mehr des Friedel-Crafts-Katalysators zugesetzt werden, jedoch bieten solche hohen Mengen im allgemeinen keinen Vorteil, bringen aber gegebenenfalls bei der Aufarbeitung Schwierig keiten. Üblicherweise wird der Friedel-Crafts-Katalysator in einer Menge von 0,001-0,5 Gew.-%, bevorzugt 0,01-0,1 Gew.-% eingesetzt. Alle Mengenangaben sind auf die Menge des eingesetzten aromatischen Kohlenwasserstoffs bezogen.

Die erfindungsgemäß einsetzbaren Co-Katalysatoren umfassen neben den obengenannten Substanzen alle Substanzen, die unter den Reaktionsbedingungen Verbindungen oder Gemische von Verbindungen bilden können, die unter die obengenannten Formeln (II) bis (X) fallen. Das sind beispielsweise solche Verbindungen, die in dem heterocyclischen Ring ein- oder mehrfach ungesättigt sind. Weiterhin sind es offenkettige Vorstufen, die unter den erfindungsgemäßen Bedingungen den Ringschluß eingehen und damit in erfindungsgemäße Co-Katalysatoren übergehen. Weiterhin einsetzbar sind alle Substanzen, die durch Reaktion der zuvor genannten erfindungsgemäßen Co-Katalysatoren mit Chlor oder Chlorwasserstoff unter den Reaktionsbedingungen der Chlorierung gebildet werden können. Hier seien beispielsweise die Hydrochloride der obengenannten Co-Katalysatoren genannt.

Es ist weiterhin möglich, die Co-Katalysatoren in Kombination mit anderen, nicht als Co-Katalysatoren beanspruchten Elementen oder Verbindungen im erfindungsgemäßen Verfahren einzusetzen. Die Co-Katalysatoren können sowohl einzeln als auch im Gemisch mehrerer von ihnen eingesetzt werden. Die Mengen, in denen die erfindungsgemäßen Co-Katalysatoren eingesetzt werden, können in weiten Grenzen variieren. Mengen unter 0,0001 Gew.-% sind jedoch weniger vorteilhaft, da dann die co-katalytische Wirkung nachläßt. Es können sogar Mengen von 5 Gew.-% oder mehr an Co-Katalysator zugesetzt werden, jedoch bieten diese hohen Mengen im allgemeinen keinen Vorteil, verursachen aber gegebenenfalls Aufarbeitungsprobleme. Die erfindungsgemäßen Co-Katalysatoren werden daher im allgemeinen in einer Menge von 0,0001-0,5 Gew.-%, bevorzugt 0,0005-0,1 Gew.-%, besonders bevorzugt 0,0005-0,0075 Gew.-%, bezogen auf den eingesetzten aromatischen Kohlenwasserstoff, eingesetzt.

Das Molverhältnis des Gemisches aus Friedel-Crafts-Katalysator(en) und Co-Katalysator(en) kann im erfindungsgemäßen Verfahren in weiten Grenzen variiert werden. Im allgemeinen ist es vorteilhaft, den Co-Katalysator nicht in zu großem Überschuß gegenüber dem Friedel-Crafts-Katalysator einzusetzen. Ebenso ist es im allgemeinen vorteilhafter, auch den Überschuß des Friedel-Crafts-Katalysators nicht zu groß anzusetzen. Erfindungsgemäß ist ein molares Verhältnis von Friedel-Crafts-Katalysator zu Co-Katalysator von 100:1-1:10, bevorzugt 75:1-1:4, besonders bevorzugt 50:1-1:2.

Der Wassergehalt der Reaktionsmischung ist im Prinzip nicht kritisch, solange der verwendete Friedel-Crafts-Katalysator nicht völlig desaktiviert wird. Daher ist es bevorzugt, die Einsatzstoffe nicht besonders zu trocknen, sondern sie in dem Zustand zu verwenden, in dem sie üblicherweise in der Praxis der technischen Chemie vorkommen. Jedoch ist es erfindungsgemäß ebenso möglich, einige oder alle Einsatzstoffe zu trocknen. Im allgemeinen sollte der Wassergehalt nicht über der Sättigungsgrenze der Einsatzstoffe liegen. Bevorzugt beträgt der Wassergehalt der Reaktionsmischung bis zu 250 ppm, besonders bevorzugt bis zu 150 ppm, ganz besonders bevorzugt bis zu 100 ppm.

Für die praktische Durchführung des erfindungsgemäßen Verfahrens ist die Reihenfolge der Zugabe der einzelnen Komponenten des Reaktionsgemisches beliebig. Hierbei läßt sich das Verfahren sowohl kontinuierlich als auch diskontinuierlich durchführen. Eine beispielhafte Ausführungsform ist die folgende:

Der gewünschte aromatische Kohlenwasserstoff, beispielsweise Toluol, wird vorgelegt und auf die gewünschte Temperatur (beispielsweise 50° C) gebracht. Dann gibt man in beliebiger Reihenfolge die gewünschten Mengen an Friedel-Crafts-Katalysator(en) und Co-Katalysator(en) zu und leitet unter weitgehender Konstanthaltung der Temperatur Chlor gasförmig bis zum gewünschten Chlorierungsgrad ein. Anschließend wird das Gemisch in üblicher Weise durch Destillation aufgearbeitet.

Eine weitere beispielhafte Ausführungsform ist die folgende:

Man stellt eine Mischung aus Alkylbenzol mit den gewünschten Anteilen an Katalysator und Co-Katalysator her und bringt diese auf die gewünschte Reaktionstemperatur. Dann wird Chlorierungsmittel bis zum gewünschten Chlorierungsgrad eingeleitet. Die Aufarbeitung kann auch hier in üblicher Weise durch Destillation erfolgen.

Eine weitere Ausführungsform ist die folgende:

Man stellt eine Lösung von Katalysator und Co-Katalysator in dem Alkylbenzol her und führt diese einer kontinuierlich arbeitenden Chlorierapparatur zu. Man leitet ebenfalls kontinuierlich ein Chlorierungsmittel so schnell ein, daß der gewünschte Chlorierungsgrad erreicht wird. Auch hier kann die kontinuierlich anfallende Reaktionsmischung in üblicher Weise durch Destillation aufgearbeitet werden.

Im Gegensatz zum erfindungsgemäßen Verfahren hatten die bisher bekannten Heterocyclen zur Steuerung der o/p-Selektivität immer eine andere Struktur, nämlich die Form von drei linear anellierten 6-Ringen.

Beim erfindungsgemäßen Verfahren ist es überraschend, daß die erfindungsgemäßen Co-Katalysatoren eine so ausgeprägte Selektionswirkung auf das o/p-Verhältnis haben, daß überwiegend die p-Verbindung gebildet wird. Ausgesprochen überraschend und überaus vorteilhaft ist ferner die Tatsache, daß die erfindungsgemäßen Co-Katalysatoren gerade mit dem technisch außerordentlich günstigen und wünschenswerten Friedel-Crafts-Katalysator $FeCl_3$ so gute Ergebnisse liefern. Das so beispielsweise für Toluol erreichbare o/p-Verhältnis von o/p = 0,55 ist das niedrigste, das bisher beim Einsatz von Kombinationen aus Friedel-Crafts-Katalysatoren und Co-Katalysatoren erreicht wurde.

Weiterhin überraschend ist, daß diese guten Ergebnisse bei technisch sehr vorteilhaften Temperaturen, bei spielsweise im Bereich von 40-60° C, erreicht werden. Noch weiterhin überraschend ist es, daß die erfindungsgemäßen Co-Katalysatoren ihre p-selektive Wirkung bereits bei äußerst geringen Konzentrationen zeigen, so daß die notwendigen Mengen an Co-Katalysatoren besonders gering sind. So liegen sie im besonders bevorzugten Bereich von 0,0005-0,0075 Gew.-% um Zehnerpotenzen niedriger als bei den bisher bekannten Co-Katalysatoren.

Diese Tatsache ist technisch wie ökonomisch und ökologisch außerordentlich vorteilhaft.

Die folgenden Beispiele erläutern die Erfindung.

Beispiel 1

Man legte unter Rühren 100 Gew.-Teile Toluol in einem Reaktor vor, gab 0,017 Gew.-Teile $FeCl_3$ und 0,0047 Gew.-Teile des Co-Katalysators der Formel

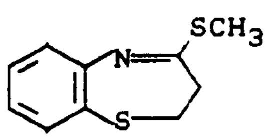

(4-Methylthio-2,3-dihydro-1,5-benzothiazepin)
zu und erhitzte auf 50° C. Unter weitgehender Konstanthaltung der Temperatur (±1° C) leitete man ca. 94 Mol-% Chlor (bezogen auf das Toluol) gasförmig im Verlauf von 5 h gleichmäßig ein. Der Restgehalt an Toluol im Reaktionsgemisch betrug 3,8 Gew.-%, das Verhältnis von ortho-Chlortoluol zu para-Chlortoluol (o/p) betrug 0,71.

Beispiel 2

Das Verfahren von Beispiel 1 wurde wiederholt. Man gab jedoch 0,0050 Gew.-Teile des Co-Katalysators der Formel

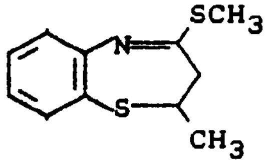

(2-Methyl-4-methylthio-2,3-dihydro-1,5-benzothiazepin)
zu. Der Resttoluolgehalt betrug 2,7 Gew.-%, das o/p-Verhältnis war 0,71.

Beispiel 3

Das Verfahren von Beispiel 1 wurde wiederholt. Man gab jedoch 0,0064 Gew.-Teile des Co-Katalysators

der Formel

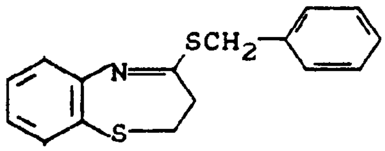

(4-Benzylthio-2,3-dihydro-1,5-benzothiazepin) zu. Das Resttoluolgehalt betrug 4,6 %, das o/p-Verhältnis war 0,86.

Beispiel 4

Das Verfahren von Beispiel 1 wurde wiederholt. Man gab jedoch 0,0045 Gew.-Teile des Co-Katalysators der Formel

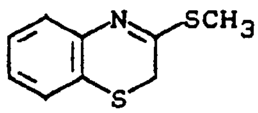

(3-Methylthio-2H-1,4-benzothiazin) zu. Der Resttoluolgehalt betrug 4,1 Gew.-%, das o/p-Verhältnis war 0,97.

Beispiel 5

Das Verfahren von Beispiel 1 wurde wiederholt. Man gab jedoch 0,0050 Gew.-Teile des Co-Katalysators der Formel

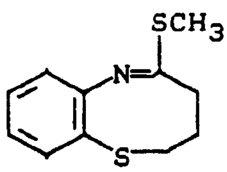

(5-Methylthio-3,4-dihydro-2H-1,6-benzothiazocin) zu. Der Resttoluolgehalt betrug 3,5 Gew.-%, das o/p-Verhältnis war 0,93.

Beispiel 6

Das Verfahren von Beispiel 1 wurde wiederholt. Man gab jedoch 0,0055 Gew.-Teile des Co-Katalysators der Formel

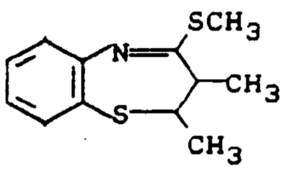

(2,3-Dimethyl-4-methylthio-2,3-dihydro-1,5-benzothiazepin) zu. Der Resttolulgehalt betrug 4,1 Gew.-%, das o/p-Verhältnis war 0,66.

Beispiel 7

Das Verfahren von Beispiel 1 wurde wiederholt. Man gab jedoch 0,0059 Gew.-Teile des Co-Katalysators der Formel

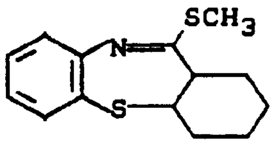

(2,3-Tetramethylen-4-methylthio-2,3-dihydro-1,5-benzothiazepin)
zu. Der Resttoluolgehalt betrug 4,0 Gew.-%, das o/p-Verhältnis war 0,63.

Beispiel 8

Das Verfahren von Beispiel 1 wurde wiederholt. Man legte jedoch 100 Gew.-Teile Ethylbenzol vor und gab 0,0060 Gew.-Teile des Co-Katalysators von Beispiel 7 zu. Der Restgehalt an Ethylbenzol betrug 10,7 Gew.-%; das Verhältnis von ortho-Chlorethylbenzol zu para-Chlorethylbenzol betrug 0,47.

Beispiel 9

Das Verfahren von Beispiel 1 wurde wiederholt. Man legte jedoch 100 Gew.-Teile Isopropylbenzol vor und gab 0,0060 Gew.-Teile des Co-Katalysators von Beispiel 7 zu. Der Restgehalt an Isopropylbenzol betrug 10,9 Gew.-%, das Verhältnis von ortho-Chlorisopropylbenzol zu para-Chlorisopropylbenzol betrug 0,24.

Beispiel 10

Das Verfahren von Beispiel 1 wurde wiederholt. Man legte jedoch 100 Gew.-Teile t-Butylbenzol vor und gab 0,0059 Gew.-Teile des Co-Katalysatos von Beispiel 7 zu. Der Restgehalt an t-Butylbenzol betrug 9,4 Gew.-%, das Verhältnis von ortho-Chlor-t-butylbenzol zu para-Chlor-t-butylbenzol betrug 0,11.

Beispiel 11

Das Verfahren von Beispiel 1 wurde wiederholt. Man legte jedoch 100 Gew.-Teile Cyclohexylbenzol vor und gab 0,0055 Gew.-Teile des Co-Katalysators von Beispiel 6 zu. Der Restgehalt an Cyclohexylbenzol betrug 10,57 Gew.-%, das Verhältnis von ortho-Chlor-cyclohexylbenzol zu p-Chlor-cyclohexylbenzol betrug 0,23.

Beispiel 12

Das Verfahren von Beispiel 1 wurde wiederholt. Man gab jedoch 0,0059 Gew.-Teile des Co-Katalysators der Formel

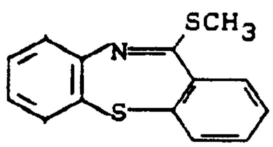

(4-Methylthio-1,5-dibenzo-[b,f]-thiazepin)
zu. Der Resttoluolgehalt betrug 4,1 Gew.-%, das o/p-Verhältnis war 1,26.

Beispiel 13

Das Verfahren von Beispiel 1 wurde wiederholt. Man gab jedoch 0,0060 Gew.-Teile des Co-Katalysators der Formel

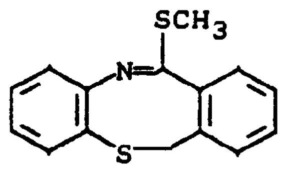

(5-Methylthio-2H-1,6-dibenzo-[c,g]-thiazocin)
zu. Der Resttoluolgehalt betrug 5,1 Gew.-%, das o/p-Verhältnis war 1,16.

Beispiel 14

Das Verfahren von Beispiel 1 wurde wiederholt. Man gab jedoch 0,0055 Gew.-Teile des Co-Katalysators der Formel

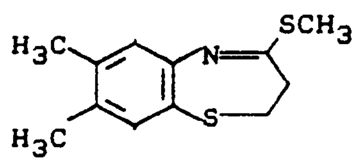

(4-Methylthio-7,8-dimethyl-2,3-dihydro-1,5-benzothiazepin)
zu. Der Resttoluolgehalt betrug 2,9 Gew.-%, das o/p-Verhältnis war 0,68.

Beispiel 15

Das Verfahren von Beispiel 1 wurde wiederholt. Man gab jedoch 0,0065 Gew.-Teile des Co-Katalysators der Formel

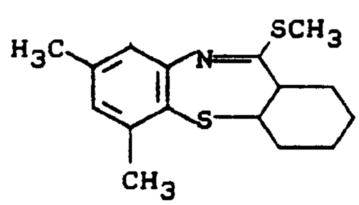

(7,9-Dimethyl-4-methylthio-2,3-tetramethylen-2,3-dihydro-1,5-benzothiazepin)
zu. Der Resttoluolgehalt betrug 3,7 Gew.-%, das o/p-Verhältnis war 0,55.

Beispiel 16

Das Verfahren von Beispiel 1 wurde wiederholt. Man gab jedoch 0,0055 Gew.-Teile des Co-Katalysators der Formel

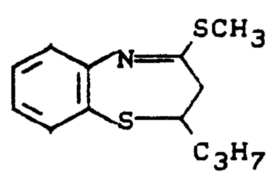

(2-Propyl-4-methylthio-2,3-dihydro-1,5-benzothiazepin)
zu. Der Resttoluolgehalt betrug 2,8 Gew.-%, das o/p-Verhältnis war 0,69.

Beispiel 17

Das Verfahren von Beispiel 1 wurde wiederholt. Man gab jedoch 0,0064 Gew.-Teile des Co-Katalysators der Formel

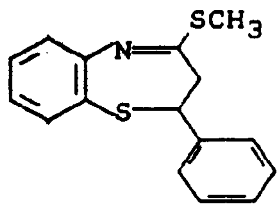

(2-Phenyl-4-methylthio-2,3-dihydro-1,5-benzothiazepin)
zu. Der Resttoluolgehalt betrug 4,7 Gew.-%, das o/p-Verhältnis war 1,06.

Beispiel 18

Das Verfahren von Beispiel 1 wurde wiederholt. Man gab jedoch 0,0062 Gew.-Teile des Co-Katalysators der Formel

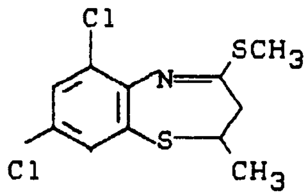

(6,8-Dichlor-4-methylthio-2,3-dihydro-1,5-benzothiazepin)
zu. Der Resttoluolgehalt betrug 3,75 Gew.-%, das o/p-Verhältnis war 0,91.

Beispiel 19

Das Verfahren von Beispiel 1 wurde wiederholt. Man gab jedoch 0,0061 Gew.-Teile des Co-Katalysators der Formel

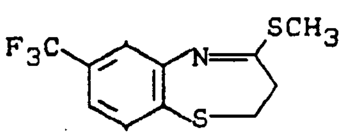

(7-Trifluormethyl-4-methylthio-2,3-dihydro-1,5-benzothiazepin)
zu. Der Resttoluolgehalt betrug 4,8 Gew.-%, das o/p-Verhältnis war 0,84.

Beispiel 20

Das Verfahren von Beispiel 1 wurde wiederholt. Man gab jedoch 0,0052 Gew.-Teile des Co-Katalysators der Formel

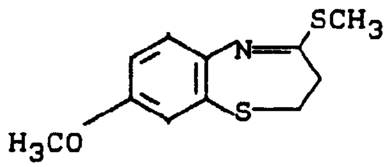

(8-Methoxy-4-methylthio-2,3-dihydro-1,5-benzothiazepin)
zu. Der Resttoluolgehalt betrug 2,1 Gew.-%, das o/p-Verhältnis war 0,78.

Beispiel 21

Das Verfahren von Beispiel 1 wurde wiederholt. Man gab jedoch 0,0045 Gew.-Teile des Co-Katalysators der Formel

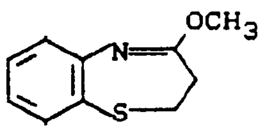

(4-Methoxy-2,3-dihydro-1,5-benzothiazepin)
zu. Der Resttoluolgehalt betrug 3,9 Gew.-%, das o/p-Verhältnis war 0,71.

Beispiel 22

Das Verfahren von Beispiel 1 wurde wiederholt. Man gab jedoch 0,0065 Gew.-Teile des Co-Katalysators der Formel

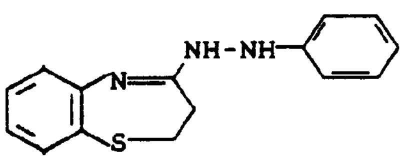

(4-Phenylhydrazino-2,3-dihydro-1,5-benzothiazepin)
zu. Der Resttoluolgehalt betrug 4,2 Gew.-%, das o/p-Verhältnis war 0,79.

Beispiel 23

Das Verfahren von Beispiel 1 wurde wiederholt. Man gab jedoch 0,0040 Gew.-Teile des Co-Katalysators der Formel

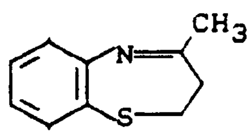

(4-Methyl-2,3-dihydro-1,5-benzothiazepin)
zu. Der Resttoluolgehalt betrug 3,7 Gew.-%, das o/p-Verhältnis war 0,75.

Beispiel 24

Das Verfahren von Beispiel 1 wurde wiederholt. Man gab jedoch 0,0045 Gew.-Teile des Co-Katalysators der Formel

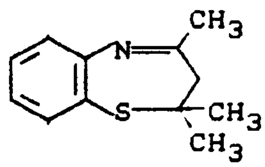

(2,2,4-Trimethyl-2,3-dihydro-1,5-benzothiazepin)
zu. Der Resttoluolgehalt betrug 4,5 Gew.-%, das o/p-Verhältnis war 1,00.

Beispiel 25

Das Verfahren von Beispiel 1 wurde wiederholt. Man gab jedoch 0,0050 Gew.-Teile des Co-Katalysators der Formel

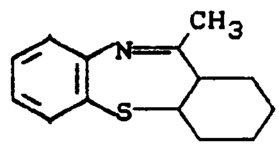

(4-Methyl-2,3-tetramethylen-2,3-dihydro-1,5-benzothiazepin)
zu. Der Resttoluolgehalt betrug 3,4 Gew.-%, das o/p-Verhältnis war 0,68.

Beispiel 26

Es wurde eine Lösung aus 100 Gew.-Teilen Toluol, 0,017 Gew.-Teilen $FeCl_3$ und 0,0055 Gew.-Teilen des Co-Katalysators aus Beispiel 7 bei Raumtemperatur hergestellt. Diese Lösung führte man einem bei 40 bis 45° C kontinuierlich arbeitenden Chlorierreaktor zu, wobei gleichzeitig die äquivalente Menge an Chlorierungsprodukt entnommen wurde. Als Chlorierungsmittel wurde gasförmiges Chlor so schnell zugeführt, daß der Umsatz weitgehend konstant bei ca. 94 Mol-% lag. Das abgeführte Chloriergemisch enthielt ca. 7,5 Gew.-% Toluol. Das Verhältnis von ortho-Chlortoluol zu para-Chlortoluol war 0,63.

Beispiel 27 (Vergleichsbeispiel)

In 100 Gew.-Teilen Toluol wurden 0,07 Gew.-Teile $FeCl_3$ und 0,29 Gew.-Teile des nach EP 0 173 222 hergestellten Phenoxathiinderivats gelöst. Man leitete bei 50° C ca. 94 Mol-% Chlor gasförmig unter Rühren ein. Der Restgehalt an Toluol betrug 7,9 %, das o/p-Verhältnis 0,88.

Beispiel 28 (Vergleichsbeispiel)

Das Verfahren des Beispiels 27 wurde wiederholt. In 100 Gew.-Teilen Toluol wurden 0,017 Gew.-Teile $FeCl_3$ und 0,008 Gew.-Teile des nach EP 0 173 222 hergestellten Phenoxathiinderivats gelöst. Man leitete bei 50° C ca. 94 Mol-% $Cl_2$ gasförmig unter Rühren ein. Der Restgehalt an Toluol betrug 6,4 Gew.-%, das o/p-Verhältnis 1,26.

Beispiel 29 (Vergleichsbeispiel)

Das Verfahren des Beispiels 27 wurde wiederholt. In 100 Gew.-Teilen Toluol wurden 0,017 Gew.-Teile $FeCl_3$ und 0,0065 Gew.-Teile des in Beispiel 4 von US 4 031 147 genannten Co-Katalysators der Formel

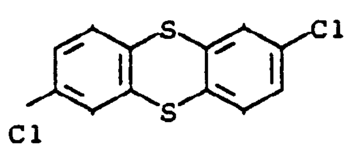

(2,7-Dichlorthianthren)
gelöst. Man erhitzte auf 50° C und leitete unter Rühren ca. 94 Mol-% $Cl_2$ gasförmig ein, Der Resttoluolgehalt betrug 6,7 Gew.-%, das o/p-Verhältnis 1,55.

Beispiel 30 (Vergleichsbeispiel)

Das Verfahren des Beispiels 27 wurde wiederholt. In 100 Gew.-Teilen Toluol wurden 0,017 Gew.-Teile $FeCl_3$ und 0,006 Gew.-Teile des in Beispiel 1 von EP 0 126 669 genannten Co-Katalysators der Formel

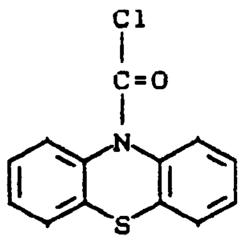

(N-Chlorcarbonylphenothiazin)
gelöst. Man erhitzte auf 50° C und leitete unter Rühren ca. 94 Mol-% Chlor gasförmig ein. Der Resttoluolgehalt betrug 5,6 Gew.-%, das o/p-Verhältnis 1,04.

Beispiel 31

Man legte unter Rühren 100 Gew.-Teile Toluol in einem Reaktor vor und gab 0,017 Gew.-Teile $FeCl_3$ und 0,0050 Gew.-Teile des Co-Katalysators der Formel

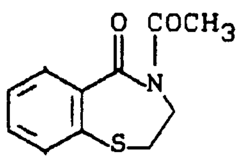

(4-Acetyl-2,3-dihydro-1,4-benzothiazepin-5(4H)-on)
zu und erhitzte auf 50° C. Unter weitgehender Konstanthaltung der Temperatur (± 1° C) leitete man ca. 94 Mol.-% Chlor (bezogen auf das Alkylbenzol) gasförmig im Verlaufe von 5 Stunden gleichmäßig ein. Der Resttoluolgehalt im Reaktionsgemisch betrug 3,0 Gew.-%, das Verhältnis von ortho-Chlortoluol zu para-Chlortoluol (o/p) betrug 0,71.

Beispiel 32

Das Verfahren von Beispiel 1 wurde wiederholt. Man gab jedoch 0,0043 Gew.-Teile des Co-Katalysators der Formel

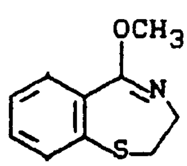

(5-Methoxy-2,3-dihydro-1,4-benzothiazepin)
zu.

Der Resttoluolgehalt betrug 4,0 Gew.-%, das o/p-Verhältnis betrug 0,83.

Beispiel 33

Das Verfahren von Beispiel 1 wurde wiederholt. Man gab jedoch 0,0049 Gew.-% des Co-Katalysators der Formel

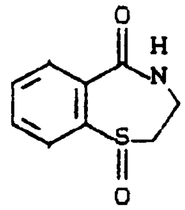

(2,3-Dihydro-1,4-benzothiazepin-5(4H)-on-1-oxid)
zu. Der Resttoluolgehalt betrug 3,1 Gew.-%, das o/p-Verhältnis war 0,70.

Beispiel 34

Das Verfahren von Beispiel 1 wurde wiederholt. Man gab jedoch 0,0045 Gew.-% des Co-Katalysators der Formel

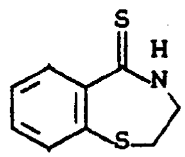

(2,3-Dihydro-1,4-benzothiazepin-5(4H)-thion)
zu. Der Resttoluolgehalt betrug 3,2 Gew.-%, das o/p-Verhältnis war 0,74.

Beispiel 35

Das Verfahren von Beispiel 1 wurde wiederholt. Man gab jedoch 0,0045 Gew.-% des Co-Katalysators der Formel

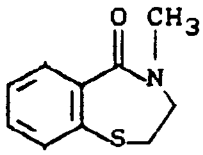

(4-Methyl-2,3-dihydro-1,4-benzothiazepin-5-(4H)-on)
zu. Der Resttoluolgehalt betrug 3,5 Gew.-%, das o/p-Verhältnis war 0,73.

Beispiel 36

Das Verfahren von Beispiel 1 wurde wiederholt. Man gab jedoch 0,0045 Gew.-% des Co-Katalysators der Formel

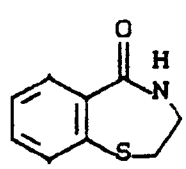

(2,3-Dihydro-1,4-benzothiazepin-5(4H)-on)
zu. Der Resttoluolgehalt betrug 3,3 Gew.-%, das o/p-Verhältnis war 0,71.

Beispiel 37

Das Verfahren von Beispiel 1 wurde wiederholt. Man gab jedoch 0,0042 Gew.-% des Co-Katalysators der Formel

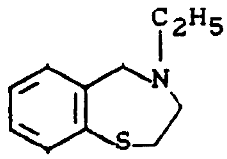

(4-Ethyl-2,3,4,5-tetrahydro-1,4-benzothiazepin)
zu. Der Resttoluolgehalt betrug 4,0 Gew.-%, das o/p-Verhältnis war 0,81.

Beispiel 38

Das Verfahren von Beispiel 1 wurde wiederholt. Man gab jedoch 0,0065 Gew.-% des Co-Katalysators der Formel

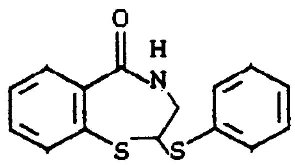

2-Phenylthio-2,3-dihydro-1,4-benzothiazepin-5(4H)-on)
zu. Der Resttoluolgehalt betrug 4,0 Gew.-%, das o/p-Verhältnis war 1,00.

Beispiel 39

Das Verfahren von Beispiel 1 wurde wiederholt. Man gab jedoch 0,0048 Gew.-% des Co-Katalysators der Formel

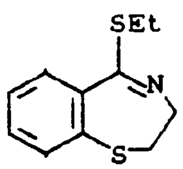

(5-Ethylthio-2,3-dihydro-1,4-benzothiazepin)
zu. Der Resttoluolgehalt betrug 3,6 Gew.-%, das o/p-Verhältnis war 0,80.

Beispiel 40

Das Verfahren von Beispiel 1 wurde wiederholt. Man gab jedoch 0,0049 Gew.-% des Co-Katalysators der Formel

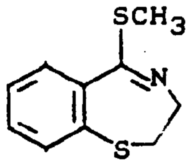

(5-Methylthio-2,3-dihydro-1,4-benzothiazepin)
zu. Der Resttoluolgehalt betrug 4,1 Gew.-%, das o/p-Verhältnis war 0,76.

Beispiel 41

Das Verfahren von Beispiel 1 wurde wiederholt. Man gab jedoch 0,0064 Gew.-% des Co-Katalysators der Formel

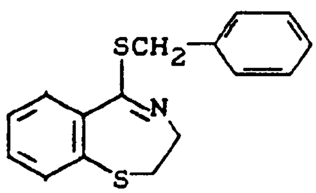

(5-Benzylthio-2,3-dihydro-1,4-benzothiazepin)
zu. Der Resttoluolgehalt betrug 3,4 Gew.-%, das o/p-Verhältnis war 0,71.

Beispiel 42

Das Verfahren von Beispiel 1 wurde wiederholt. Man gab jedoch 0,0060 Gew.-% des Co-Katalysators der Formel

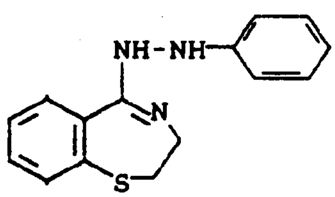

(5-β-Phenylhydrazino-2,3-dihydro-1,4-benzothiazepin)
zu. Der Resttoluolgehalt betrug 4,3 Gew.-%, das o/p-Verhältnis war 0,64.

Beispiel 43

Das Verfahren von Beispiel 1 wurde wiederholt. Man gab jedoch 0,0048 Gew.-% des Co-Katalysators der Formel

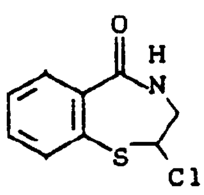

(2-Chlor-2,3-dihydro-1,4-benzothiazepin-5-(4H)-on)
zu und erhitzte auf 52 - 53° C. Der Resttoluolgehalt betrug 4,1 Gew.-%, das o/p-Verhältnis war 0,73.

Beispiel 44

Das Verfahren von Beispiel 1 wurde wiederholt. Man gab jedoch 0,0045 Gew.-% des Co-Katalysators der Formel

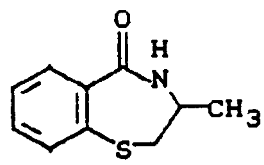

(3-Methyl-2,3-dihydro-1,4-benzothiazepin-5-(4H)-on)
zu und erhitzte auf 50 - 52°C. Der Resttoluolgehalt betrug 3,0 Gew.-%, das o/p-Verhältnis war 0,69.

Beispiel 45

Das Verfahren von Beispiel 1 wurde wiederholt. Man gab jedoch 0,0044 Gew.-% des Co-Katalysators der Formel

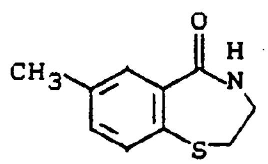

(7-Methyl-2,3-dihydro-1,4-benzothiazepin-5-(4H)-on)
zu. Der Resttoluolgehalt betrug 2,9 Gew.-%, das o/p-Verhältnis war 0,68.

Beispiel 46

Das Verfahren von Beispiel 1 wurde wiederholt. Man gab jedoch 0,0045 Gew.-% des Co-Katalysators der Formel

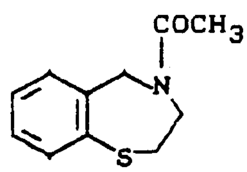

(4-Acetyl-2,3,4,5-tetrahydro-1,4-benzothiazepin)
zu. Der Resttoluolgehalt betrug 2,9 Gew.-%, das o/p-Verhältnis war 0,93.

Beispiel 47

Das Verfahren von Beispiel 1 wurde wiederholt. Man gab jedoch 0,0047 Gew.-% des Co-Katalysators der Formel

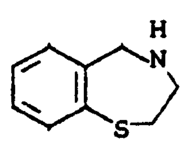

(2,3,4,5-Tetrahydro-1,4-benzothiazepin)

zu.Der Resttoluolgehalt betrug 4,3 Gew.-%, das o/p-Verhältnis war 0,88.

Beispiel 48

Das Verfahren von Beispiel 1 wurde wiederholt. Man legte jedoch 100 Gew.-Teile Ethylbenzol vor und gab 0,0043 Gew.-Teile des Co-Katalysators von Beispiel 15 zu. Der Restgehalt an Ethylbenzol betrug 4,6 Gew.-%, das Verhältnis von ortho-Chlorethylbenzol zu para-Chlorethylbenzol betrug 0,50.

Beispiel 49

Das Verfahren von Beispiel 1 wurde wiederholt. Man legte jedoch 100 Gew.-Teile an Isopropylbenzol vor und gab 0,0040 Gew.-Teile des Co-Katalysators von Beispiel 6 zu. Der Restgehalt an Isopropylbenzol betrug 5,0 Gew.-%, das Verhältnis von ortho-Chlorisopropylbenzol zu para-Chlorisopropylbenzol betrug 0,25.

Beispiel 50

Das Verfahren von Beispiel 1 wurde wiederholt. Man legte jedoch 100 Gew.-Teile an t-Butylbenzol vor und gab 0,0042 Gew.-Teile des Co-Katalysators von Beispiel 14 zu. Der Restgehalt an t-Butylbenzol betrug 5,2 Gew.-%, das Verhältnis von ortho-Chlor-tert-butylbenzol zu para-Chlor-tert-butylbenzol betrug 0,16.

Beispiel 51

Das Verfahren von Beispiel 1 wurde wiederholt. Man legte jedoch 100 Gew.-Teile an Cyclohexylbenzol vor und gab 0,0042 Gew.-Teile des Co-Katalysators von Beispiel 5 zu. Der Restgehalt an Cyclohexylbenzol betrug 5,5 Gew.-%, das Verhältnis von ortho-Chlor-cyclohexylbenzol zu p-Chlor-cyclohexylbenzol betrug 0,22.

Beispiel 52

Das Verfahren von Beispiel 1 wurde wiederholt. Man legte jedoch 0,0050 Gew.-Teile eines Kaliumsalzes folgender Formel zu

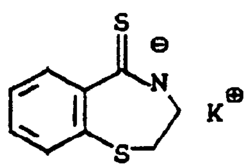

(2,3-Dihydro-1,4-benzothiazepin-5-(4H)-thion-K-salz) ,
das während der Chlorierung in den Co-Katalysator von Beispiel 4 übergeht. Der Resttoluolgehalt betrug 3,8 Gew.-%, das o/p-Verhältnis war 0,73.

Beispiel 53

Das Verfahren von Beispiel 1 wurde wiederholt. Man gab jedoch 0,0055 Gew.-Teile des Co-Katalysators der Formel

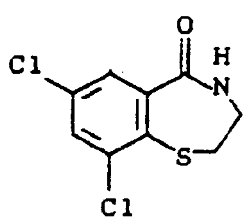

(7,9-Dichlor-2,3-dihydro-1,4-benzothiazepin-5-(4H)-on)
zu. Der Resttoluolgehalt betrug 4,5 Gew.-%, das o/p-Verhältnis war 0,74.

Beispiel 54

Das Verfahren von Beispiel 1 wurde wiederholt. Man gab jedoch 0,0050 Gew.-Teile des Co-Katalysators der Formel

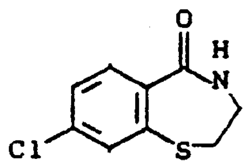

(8-Chlor-2,3-dihydro-1,4-benzothiazepin-5-(4H)-on)
zu. Der Resttoluolgehalt betrug 3,8 Gew.-%, das o/p-Verhältnis war 0,74.

Beispiel 55

Das Verfahren von Beispiel 1 wurde wiederholt. Man gab jedoch 0,0048 Gew.-Teile des Co-Katalysators der Formel

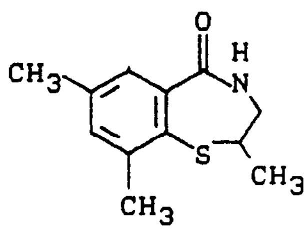

(2,7,9-Trimethyl-2,3-dihydro-1,4-benzothiazepin-5-(4H)-on)
zu. Der Resttoluolgehalt betrug 4,9 Gew.-%, das o/p-Verhältnis war 0,66.

Beispiel 56

Das Verfahren von Beispiel 1 wurde wiederholt. Man gab jedoch 0,0055 Gew.-Teile des Co-Katalysators der Formel

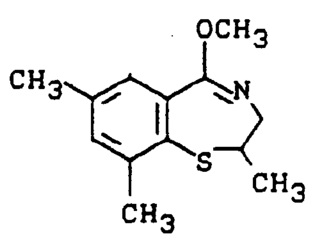

(2,7,9-Trimethyl-5-methoxy-2,3-dihydro-1,4-benzothiazepin)
zu. Der Restgehalt an Toluol betrug 3,0 Gew.-%, das o/p-Verhältnis war 0,66.

Beispiel 57

Das Verfahren von Beispiel 1 wurde wiederholt. Man gab jedoch 0,00073 Gew.-Teile des Co-Katalysators der Formel

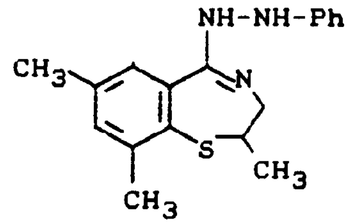

(2,7,9-Trimethyl-5-β-phenylhydrazino-2,3-dihydro-1,4-benzothiazepin) zu. Der Restgehalt an Toluol war 3,3 Gew.-%, das o/p-Verhältnis betrug 0,65.

**Patentansprüche**

1. Verfahren zur Kernchlorierung von aromatischen Kohlenwasserstoffen der Formel

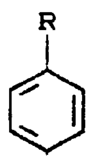 (I) ,

worin
R geradkettiges oder verzweigtes $C_1$-$C_{12}$-Alkyl oder $C_3$-$C_8$-Cycloalkyl bedeutet,
in Gegenwart von Friedel-Crafts-Katalysatoren und in Gegenwart von Co-Katalysatoren in flüssiger Phase, dadurch gekennzeichnet, daß man als Co-Katalysatoren cyclische benzokondensierte Imine der Formel

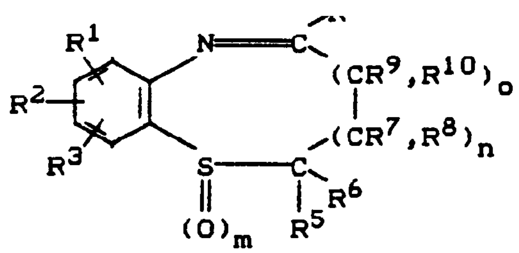 (II) ,

in der
$R^1$ und $R^2$ unabhängig voneinander Wasserstoff, Hydroxy, Amino, Cyano, Halogen, Nitro, Carboxyl, Halogenocarbonyl, Carboxyamid, Alkoxycarbonyl, Alkyl, Aryl, Alkoxy, Aryloxy, Acyloxy, Alkylthio, Arylthio, Acylthio, Acyl, Thioacyl oder Acylamino bedeuten,
$R^3$ für Wasserstoff oder Chlor steht und weiterhin mit einem der Reste $R^1$ oder $R^2$ bei benachbarter Substitution und gemeinsam mit den substituierten C-Atomen einen anellierten gesättigten, ungesättigten oder aromatischen isocyclischen oder heterocyclischen 5-8-Ring bilden kann,
$R^4$ Wasserstoff, Alkyl, Aryl, Halogen, Alkylthio, Arylthio, Alkoxy, Aryloxy, Amino, Hydrazino, Alkylhydrazino oder Phenylhydrazino bedeutet,
m, n und o unabhängig voneinander den Wert 0 oder 1 annehmen können,
$R^5$, $R^7$ und $R^9$ unabhängig voneinander Wasserstoff, Alkyl, Alkoxy, Phenyl, Acyloxy, Cyano, Halogen, Carboxyl, Alkoxycarbonyl, Phenoxy oder Acyl bedeuten und
$R^6$, $R^8$ und $R^{10}$ unabhängig voneinander Wasserstoff, Alkyl oder Halogen bedeuten, wobei $R^5$ und $R^7$ oder $R^7$ und $R^9$ gemeinsam mit den substituierten C-Atomen einen gesättigten, ungesättigten oder aromatischen isocyclischen oder heterocyclischen 5-8-Ring darstellen können und wobei
weiterhin $R^6$ und $R^8$ oder $R^8$ und $R^{10}$ gemeinsam eine Doppelbindung bilden können und wobei
weiterhin $R^5$ und $R^6$ gemeinsam doppelt gebundene Sauerstoff, Schwefel oder $R^{11}$-substituierten Stickstoff darstellen können, wobei $R^{11}$ Alkyl, Aryl, Acyl, Alkylamino oder Arylamino bedeutet,
oder Benzo[f]-1,4-thiazepine der Formel

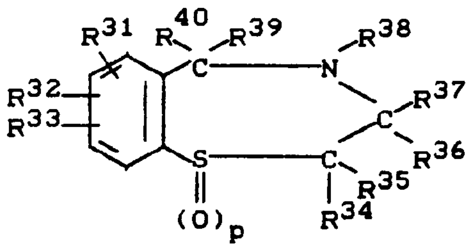 (III) ,

in der
$R^{31}$ und $R^{32}$ unabhängig voneinander Wasserstoff, Hydroxy, Amino, Cyano, Halogen, Nitro, $C_1$-$C_8$-Alkyl, nicht substituiertes oder durch $R^{31}$ und $R^{32}$ substituiertes Phenyl (mit Ausnahme der erneuten Substitution durch $R^{31}$- und $R^{32}$-substituiertes Phenyl), $C_1$-$C_8$-Alkoxy, Phenoxy, $C_1$-$C_8$-Acyloxy, $C_1$ $C_8$-Acyl oder $C_1$-$C_8$-Alkoxycarbonyl bedeuten,
$R^{33}$ für Wasserstoff oder Chlor steht und weiterhin mit einem der Reste $R^{31}$ oder $R^{32}$ und gemeinsam mit den substituierten C-Atomen einen anellierten gesättigten, ungesättigten oder aromatischen isocyclischen oder heterocyclischen 5-8-Ring bilden kann,
$R^{34}$, $R^{36}$ und $R^{40}$ unabhängig voneinander Wasserstoff, $C_1$-$C_8$-Alkyl, nicht substituiertes oder durch $R^{31}$ und $R^{32}$ substituiertes Phenyl (mit Ausnahme der erneuten Substitution durch $R^{31}$- und $R^{32}$-substituiertes Phenyl), $C_1$-$C_8$-Acyl, $C_1$-$C_8$-Alkoxycarbonyl, Cyano, Halogen, Carboxyl, $C_1$-$C_8$-Alkoxy, $C_1$-$C_8$-Alkylthio, Phenylthio, Benzylthio, Phenoxy oder $C_1$-$C_8$-Acyloxy bedeuten,
$R^{35}$, $R^{37}$ und $R^{39}$ unabhängig voneinander Wasserstoff, $C_1$-$C_8$-Alkyl, Halogen, $C_1$-$C_8$-Alkoxy oder $C_1$-$C_8$-Alkylthio bedeuten,
$R^{38}$ Wasserstoff, $C_1$-$C_8$-Alkyl, nicht substituierte oder durch $R^{31}$- und $R^{32}$-substituiertes Phenyl (mit Ausnahme der erneuten Substitution durch $R^{31}$- und $R^{32}$-substituiertes Phenyl), $C_1$-$C_8$-Acyl, $C_1$-$C_8$-Thioacyl, Halogencarbonyl oder $C_1$-$C_8$-Alkoxycarbonyl bedeutet und
p für die Zahl Null oder Eins steht,
wobei weiterhin
die Substituentenpaare $R^{34}$ und $R^{35}$, $R^{36}$ und $R^{37}$ sowie $R^{39}$ und $R^{40}$ unabhängig voneinander doppelt gebundenen Sauerstoff, Schwefel oder $R^{38}$-substituierten Stickstoff bedeuten können und wobei weiterhin
die Substituentenpaare $R^{35}$ und $R^{36}$ sowie $R^{38}$ und $R^{39}$ unabhängig voneinander eine Doppelbindung bilden können und wobei weiterhin
die Substituentenpaare $R^{34}$ und $R^{37}$ sowie $R^{38}$ und $R^{39}$ unabhängig voneinander 3- bis 5-gliedriges Alkylen bilden können, bei dem 1 oder 2 C-Atome durch Sauerstoff, Schwefel oder $R^{38}$-substituierten Stickstoff ersetzt sein können, und wobei weiterhin
$R^{40}$ auch die Bedeutung Hydrazino, $C_1$-$C_8$-Alkylhydrazino oder Phenyl-hydrazino annehmen kann, einsetzt.

2. Verfahren nach Anspruch 1, dadurch gekennzeichnet, daß man cyclische benzokondensierte Imine der Formeln

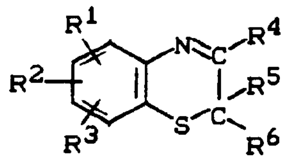 (IV) ,

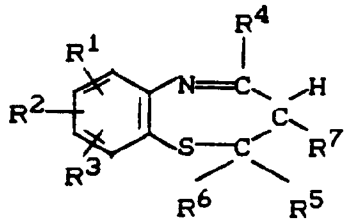 (V)

und/oder

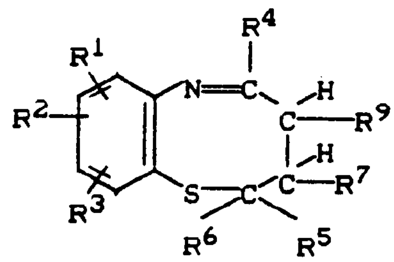

(VI)

einsetzt, in denen $R^1$ bis $R^9$ den in Anspruch 1 genannten Bedeutungsumfang haben.

3. Verfahren nach Anspruch 2, dadurch gekennzeichnet, daß an die Stelle von $R^1$, $R^2$ und $R^3$ die Reste $R^{12}$, $R^{13}$ und $R^{14}$ treten, von denen
$R^{12}$ und $R^{13}$ unabhängig voneinander Wasserstoff, Halogen, Nitro, Alkyl, Aryl, Alkoxy, Aryloxy, Acyloxy, Alkylthio, Arylthio, Acyl oder Thioacyl bedeuten und
$R^{14}$ für Wasserstoff oder Chlor steht und weiterhin mit einem der Reste $R^{12}$ und $R^{13}$ und gemeinsam mit den durch sie substituierten C-Atomen einen anellierten, gesättigten isocyclischen 5-7-Ring oder einen anellierten Benzolring bilden kann,
und bevorzugt an die Stelle von $R^{12}$, $R^{13}$ und $R^{14}$ die Reste $R^{21}$, $R^{22}$ und $R^{23}$ treten, von denen
$R^{21}$ und $R^{22}$ unabhängig voneinander Wasserstoff, $C_1$-$C_4$-Alkyl, $C_1$-$C_4$-Alkoxy, Fluor oder Chlor bedeuten und
$R^{23}$ für Wasserstoff oder Chlor steht und weiterhin mit einem der Reste $R^{21}$ oder $R^{22}$ und gemeinsam mit den durch sie substituierten C-Atomen einen anellierten Cyclopentan-, Cyclohexan- oder Benzolring bilden kann.

4. Verfahren nach Anspruch 2, dadurch gekennzeichnet, daß an die Stelle von $R^4$ der Rest $R^{15}$ tritt, der Wasserstoff, $C_1$-$C_4$-Alkyl, $C_1$-$C_4$-Alkoxy, Phenyl, Benzyl, Chlor, $C_1$-$C_4$-Acyl, $C_1$-$C_4$-Alkylthio, Benzyloxy, Benzylthio, Hydrazino, $C_1$-$C_4$-Alkyl-hydrazino oder Phenyl-hydrazino bedeutet,
und bevorzugt an die Stelle von $R^{15}$ der Rest $R^{24}$ tritt, der Wasserstoff, $C_1$-$C_4$-Alkyl, Benzyl, Phenyl, $C_1$-$C_4$-Alkoxy, $C_1$-$C_4$-Alkylthio, Benzyloxy, Benzylthio, Hydrazino oder Phenyl-hydrazino bedeutet.

5. Verfahren nach Anspruch 2, dadurch gekennzeichnet, daß an die Stelle von $R^5$, $R^6$, $R^7$ und $R^9$ die Reste $R^{16}$, $R^{17}$, $R^{18}$ und $R^{19}$ treten, die unabhängig voneinander Wasserstoff, $C_1$-$C_4$-Alkyl, Fluor oder Chlor bedeuten, wobei $R^{16}$, $R^{18}$ und $R^{19}$ zusätzlich $C_1$-$C_4$-Alkoxy, $C_1$-$C_4$-Acyl oder Phenyl bedeuten können und weiterhin bei benachbarter Substitution gemeinsam mit den substituierten C-Atomen einen Cyclopentan-Cyclohexan- oder Benzolring bilden können.

6. Verfahren nach Anspruch 2, dadurch gekennzeichnet, daß cyclische benzokondensierte Imine der Formel

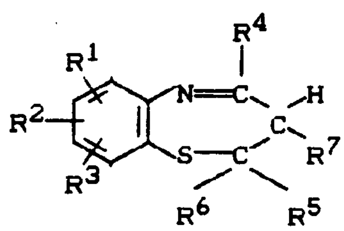

(V)

eingesetzt werden, in denen $R^1$ bis $R^7$ den in Anspruch 2 genannten Umfang haben.

7. Verfahren nach Anspruch 1, dadurch gekennzeichnet, daß man als Co-Katalysatoren Benzo[f]-1,4-thiazepine der Formel

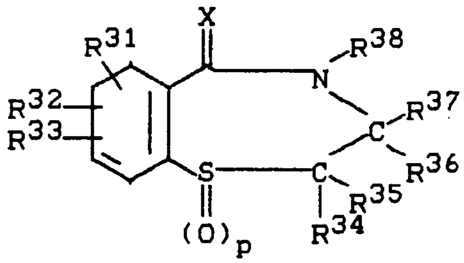

(VII)

einsetzt, in der
X doppelt gebundenen Sauerstoff, Schwefel oder $R^{38}$-substituierten Stickstoff bedeutet und
$R^{31}$, $R^{32}$, $R^{33}$, $R^{34}$, $R^{35}$, $R^{36}$, $R^{37}$, $R^{38}$ und p den in Anspruch 1 genannten Bedeutungsumfang haben.

8. Verfahren nach Anspruch 1, dadurch gekennzeichnet, daß man Co-Katalysatoren der Formel

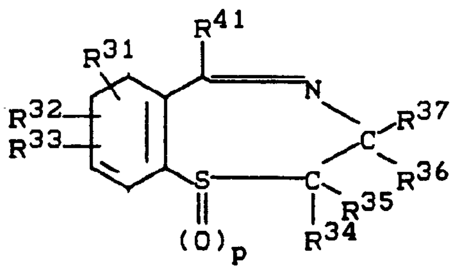

(VIII)

einsetzt, in der
$R^{41}$ für $C_1$-$C_8$-Alkyl, $C_1$-$C_8$-Alkoxy, $C_1$-$C_8$-Alkylthio, Hydrazino, β-Phenyl-hydrazino oder β-$C_1$-$C_8$-Alkyl-hydrazino steht und
$R^{31}$, $R^{32}$, $R^{33}$, $R^{34}$, $R^{35}$, $R^{36}$, $R^{37}$ und p den in Anspruch 1 genannten Bedeutungsumfang haben.

9. Verfahren nach Anspruch 1, dadurch gekennzeichnet, daß man Co-Katalysatoren der Formel

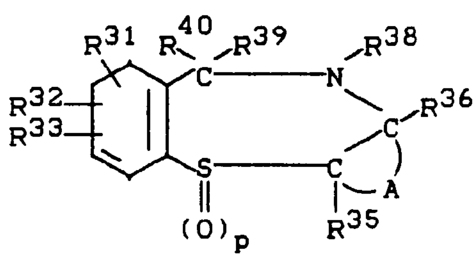

(IX)

einsetzt, in der
A Trimethylen, Tetramethylen oder Pentamethylen bedeutet und
$R^{31}$, $R^{32}$, $R^{33}$, $R^{35}$, $R^{36}$, $R^{38}$, $R^{39}$, $R^{40}$ und p den in Anspruch 1 genannten Bedeutungsumfang haben.

10. Verfahren nach Anspruch 1, dadurch gekennzeichnet, daß p den Wert Null annimmt.

11. Verfahren nach Anspruch 1, dadurch gekennzeichnet, daß an die Stelle von $R^{31}$, $R^{32}$ und $R^{33}$ die Reste $R^{42}$, $R^{43}$ bzw. $R^{44}$ treten, von denen
$R^{42}$ und $R^{43}$ unabhängig voneinander Wasserstoff, Halogen Nitro, Alkyl, Aryl, Alkoxy, Aryloxy, Acyloxy, Alkylthio, Arylthio, Acyl oder Thioacyl bedeuten und
$R^{44}$ für Wasserstoff oder Chlor steht und weiterhin mit einem der Reste $R^{42}$ und $R^{43}$ und gemeinsam mit den substituierten C-Atomen einen anellierten gesättigten isocyclischen 5-7-Ring oder einen anellierten Benzolring bilden kann,
und bevorzugt dadurch gekennzeichnet, daß an die Stelle von $R^{42}$, $R^{43}$ und $R^{44}$ die Reste $R^{51}$, $R^{52}$ bzw. $R^{53}$ treten, von denen
$R^{51}$ und $R^{52}$ unabhängig voneinander Wasserstoff, $C_1$-$C_4$-Alkyl, $C_1$-$C_4$-Alkoxy, Fluor oder Chlor bedeuten und
$R^{53}$ für Wasserstoff oder Chlor steht und weiterhin mit einem der Reste $R^{51}$ oder $R^{52}$ und gemeinsam mit den substituierten C-Atomen einen anellierten Cyclopentan-, Cyclohexan- oder Benzolring bilden kann.

12. Verfahren nach Anspruch 1, dadurch gekennzeichnet, daß die Menge an eingesetztem Co-Katalysator

0,0001-0,5 Gew.-%, bevorzugt 0,0005-0,1 Gew.-%, besonders bevorzugt 0,0005-0,0075 Gew.-%, bezogen auf den eingesetzten aromatischen Kohlenwasserstoff, beträgt.

## Claims

1. Process for the ring chlorination of aromatic hydrocarbons of the formula

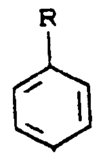

(I)

in which
R denotes straight-chain or branched $C_1$-$C_{12}$-alkyl or $C_3$-$C_8$-cycloalkyl
in the presence of Friedel-Crafts catalysts and in the presence of co-catalysts in liquid phase, characterised in that the co-catalysts used are cyclic benzo-fused imines of the formula

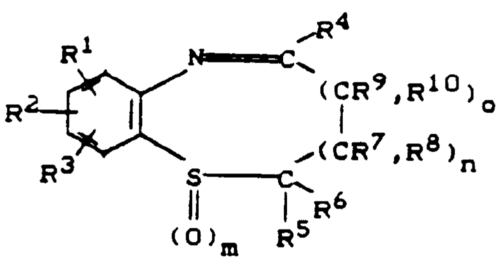

(II)

in which
$R^1$ and $R^2$, independently of one another, denote hydrogen, hydroxyl, amino, cyano, halogen, nitro, carboxyl, halogenocarbonyl, carboxyamide, alkoxycarbonyl, alkyl, aryl, alkoxy, aryloxy, acyloxy, alkylthio, arylthio, acylthio, acyl, thioacyl or acylamino,
$R^3$ represents hydrogen or chlorine and can further form a fused saturated, unsaturated or aromatic isocyclic or heterocyclic 5-8-membered ring with one of the radicals $R^1$ and $R^2$, if substituted adjacently, and together with the substituted C atoms,
$R^4$ denotes hydrogen, alkyl, aryl, halogen, alkylthio, arylthio, alkoxy, aryloxy, amino, hydrazino, alkylhydrazino or phenylhydrazino,
m, n and o, independently of one another, can adopt the value 0 or 1 and
$R^5$, $R^7$ and $R^9$, independently of one another, denote hydrogen, alkyl, alkoxy, phenyl, acyloxy, cyano, halogen, carboxyl, alkoxycarbonyl, phenoxy or acyl and
$R^6$, $R^8$ and $R^{10}$, independently of one another, denote hydrogen, alkyl or halogen, it being possible for $R^5$ and $R^7$ or $R^7$ and $R^9$ to represent a saturated, unsaturated or aromatic isocyclic or heterocyclic 5-8-membered ring together with the substituted C atoms and furthermore for
$R^6$ and $R^8$ or $R^8$ and $R^{10}$ together to form a double bond and furthermore for $R^5$ and $R^6$ to represent together doubly bound oxygen, sulphur or $R^{11}$-susbtituted nitrogen in which $R^{11}$ denotes alkyl, aryl, acyl, alkylamino or arylamino,
or benzo[f]-1,4-thiazepines of the formula

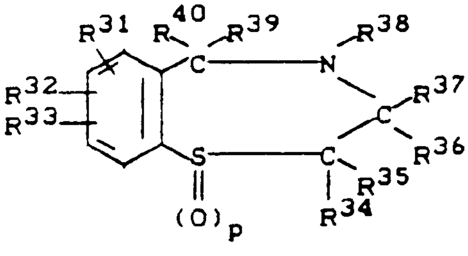

(III)

in which
$R^{31}$ and $R^{32}$, independently of one another, denote hydrogen, hydroxyl, amino, cyano, halogen, nitro, $C_1$-$C_8$-alkyl, unsubstituted or $R^{31}$- and $R^{32}$-substituted phenyl (with the exception of repeated substitution by $R^{31}$- and $R^{32}$-substituted phenyl), $C_1$-$C_8$-alkoxy, phenoxy, $C_1$-$C_8$-acyloxy, $C_1$-$C_8$-acyl or $C_1$-$C_8$-alkoxycarbonyl,
$R^{33}$ represents hydrogen or chlorine and can furthermore form a fused saturated, unsaturated or aromatic isocyclic or heterocyclic 5-8-membered ring with one of the radicals $R^{31}$ and $R^{32}$ and together with the substituted C atoms,
$R^{34}$, $R^{36}$ and $R^{40}$, independently of one another, denote hydrogen, $C_1$-$C_8$-alkyl, unsubstituted or $R^{31}$- and $R^{32}$-substituted phenyl (with the exception of repeated substitution by $R^{31}$- and $R^{32}$-substituted phenyl), $C_1$-$C_8$-acyl, $C_1$-$C_8$-alkoxycarbonyl, cyano, halogen, carboxyl, $C_1$-$C_8$-alkoxy, $C_1$-$C_8$-alkylthio, phenylthio, benzylthio, phenoxy or $C_1$-$C_8$-acyloxy,
$R^{35}$, $R^{37}$ and $R^{39}$, independently of one another, denote hydrogen, $C_1$-$C_8$-alkyl, halogen, $C_1$-$C_8$-alkoxy or $C_1$-$C_8$-alkylthio,
$R^{38}$ denotes hydrogen, $C_1$-$C_8$-alkyl, unsubstituted or $R^{31}$- and $R^{32}$-substituted phenyl (with the exception of repeated substitution by $R^{31}$- and $R^{32}$-substituted phenyl), $C_1$-$C_8$-acyl, $C_1$-$C_8$-thioacyl, halogenocarbonyl or $C_1$-$C_8$-alkoxycarbonyl and
p represents the number zero or one,
it furthermore being possible for the substituent pairs $R^{34}$ and $R^{35}$, $R^{36}$ and $R^{37}$, and $R^{39}$ and $R^{40}$, independently of one another, to denote doubly bound oxygen, sulphur or $R^{38}$-substituted nitrogen and furthermore for
the substituent pairs $R^{35}$ and $R^{36}$ and $R^{38}$ and $R^{39}$, independently of one another, to form a double bond and furthermore for
the substituent pairs $R^{34}$ and $R^{37}$, and $R^{38}$ and $R^{39}$, independently of one another, to form 3- to 5-membered alkylene in which 1 or 2 C atoms can be replaced by oxygen, sulphur or $R^{38}$-substituted nitrogen and furthermore for
$R^{40}$ also to adopt the meaning of hydrazino, $C_1$-$C_8$-alkylhydrazino or phenylhydrazino.

2. Process according to Claim 1, characterised in that cyclic benzo-fused imines of the formulae

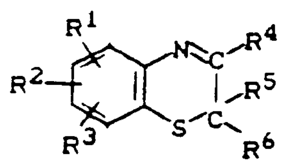

(IV),

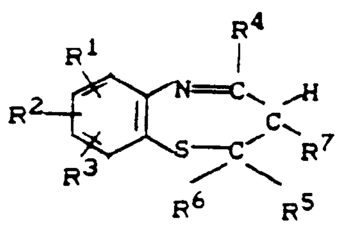

(V)

and/or

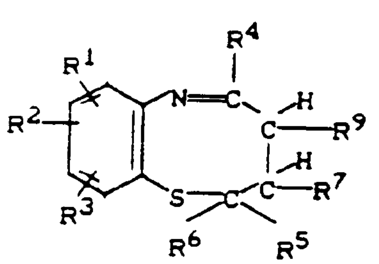

(VI)

are used in which $R^1$ and $R^9$ have the range of meanings mentioned in Claim 1.

3. Process according to Claim 2, characterised in that the radicals $R^{12}$, $R^{13}$ and $R^{14}$ take the place of $R^1$, $R^2$ and $R^3$, of which

$R^{12}$ and $R^{13}$, independently of one another, denote hydrogen, halogen, nitro, alkyl, aryl, alkoxy, aryloxy, acyloxy, alkylthio, arylthio, acyl or thioacyl and
$R^{14}$ represents hydrogen or chlorine and furthermore can form a fused, saturated isocyclic 5-7-membered ring or a fused benzene ring with one of the radicals $R^{12}$ and $R^{13}$ and together with the C atoms substituted by them, and the radicals $R^{21}$, $R^{22}$ and $R^{23}$ preferably take the place of $R^{12}$, $R^{13}$ and $R^{14}$, of which
$R^{21}$ and $R^{22}$, independently of one another, denote hydrogen, $C_1$-$C_4$-alkyl, $C_1$-$C_4$-alkoxy, fluorine or chlorine and
$R^{23}$ represents hydrogen or chlorine and furthermore can form a fused cyclopentane, cyclohexane or benzene ring with one of the radicals $R^{21}$ and $R^{22}$ and together with the C atoms substituted by them.

4. Process according to Claim 2, characterised in that the radical $R^{15}$ takes the place of $R^4$ and denotes hydrogen, $C_1$-$C_4$-alkyl, $C_1$-$C_4$-alkoxy, phenyl, benzyl, chlorine, $C_1$-$C_4$-acyl, $C_1$-$C_4$-alkylthio, benzyloxy, benzylthio, hydrazino, $C_1$-$C_4$-alkylhydrazino or phenylhydrazino, and the radical $R^{24}$ preferably takes the place of $R^{15}$ and denotes hydrogen, $C_1$-$C_4$-alkyl, benzyl, phenyl, $C_1$-$C_4$-alkoxy, $C_1$-$C_4$-alkylthio, benzyloxy, benzylthio, hydrazino or phenylhydrazino.

5. Process according to Claim 2, characterised in that the radicals $R^{16}$, $R^{17}$, $R^{18}$ and $R^{19}$ take the place of $R^5$, $R^6$, $R^7$ and $R^9$ and, independently of one another, denote hydrogen, $C_1$-$C_4$-alkyl, fluorine or chlorine, it being possible for $R^{16}$, $R^{18}$ and $R^{19}$ additionally to denote $C_1$-$C_4$-alkoxy, $C_1$-$C_4$-acyl or phenyl and furthermore to form, if substituted adjacently, a cyclopentane, cyclohexane or benzene ring together with the substituted C atoms.

6. Process according to Claim 2, characterised in that cyclic benzo-fused imines of the formula

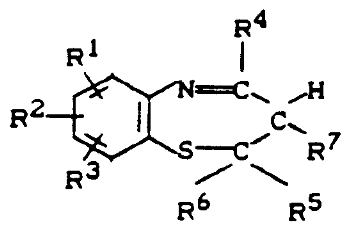

(V)

are used in which $R^1$ and $R^7$ have the range mentioned in Claim 2.

7. Process according to Claim 1, characterised in that the co-catalysts used are benzo[f]-1,4-thiazepines of the formula

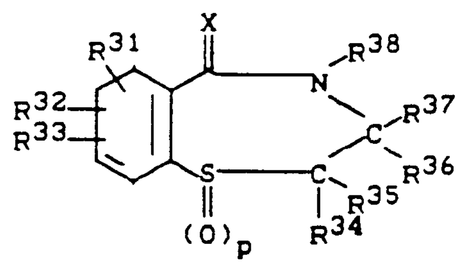

(VII)

in which
X denotes doubly bound oxygen, sulphur or $R^{38}$-substituted nitrogen and
$R^{31}$, $R^{32}$, $R^{33}$, $R^{34}$, $R^{35}$, $R^{36}$, $R^{37}$, $R^{38}$ and p have the range of meanings mentioned in Claim 1.

8. Process according to Claim 1, characterised in that co-catalysts of the formula

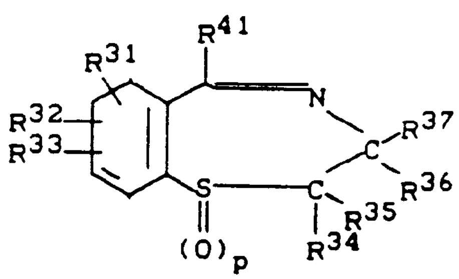

(VIII)

are used in which

$R^{41}$ represents $C_1$-$C_8$-alkyl, $C_1$-$C_8$-alkoxy, $C_1$-$C_8$-alkylthio, hydrazino, β-phenylhydrazino or β-$C_1$-$C_8$-alkylhydrazino and
$R^{31}$, $R^{32}$, $R^{33}$, $R^{34}$, $R^{35}$, $R^{36}$, $R^{37}$ and p have the range of meanings mentioned in Claim 1.

9. Process according to Claim 1, characterised in that co-catalysts of the formula

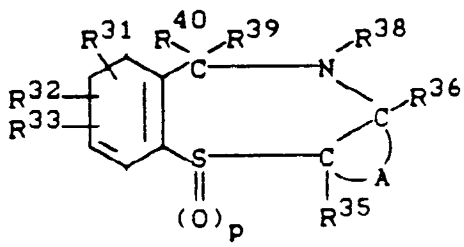

(IX)

are used in which
A denotes trimethylene, tetramethylene or pentamethylene and
$R^{31}$, $R^{32}$, $R^{33}$, $R^{34}$, $R^{35}$, $R^{36}$, $R^{37}$, $R^{40}$ and p have the range of meanings mentioned in Claim 1.

10. Process according to Claim 1, characterised in that p adopts the value zero.

11. Process according to Claim 1, characterised in that the radicals $R^{42}$, $R^{43}$ and $R^{44}$ take the place of $R^{31}$, $R^{32}$ and $R^{33}$, of which
$R^{42}$ and $R^{43}$, independently of one another, denote hydrogen, halogen, nitro, alkyl, aryl, alkoxy, aryloxy, acyloxy, alkylthio, arylthio, acyl or thioacyl and
$R^{44}$ represents hydrogen or chlorine and furthermore can form a fused saturated isocyclic 5-7-membered ring or a fused benzene ring with one of the radicals $R^{42}$ and $R^{43}$ and together with the substituted C atoms
and preferably characterised in that the radicals $R^{51}$, $R^{52}$ and $R^{53}$ take the place of $R^{42}$, $R^{43}$ and $R^{44}$, of which
$R^{51}$ and $R^{52}$, independently of one another, denote hydrogen, $C_1$-$C_4$-alkyl, $C_1$-$C_4$-alkoxy, fluorine or chlorine and
$R^{53}$ represents hydrogen or chlorine and furthermore can form a fused cyclopentane, cyclohexane or benzene ring with one of the radicals $R^{51}$ and $R^{52}$ and together with the substituted C atoms.

12. Process according to Claim 1, characterised in that the amount of co-catalyst used is 0.0001-0.5 % by weight, preferably 0.0005-0.1 % by weight, particularly preferably 0.0005-0.0075 % by weight, relative to the aromatic hydrocarbon used.

## Revendications

1. Procédé pour chlorer dans le noyau des hydrocarbures aromatiques de formule

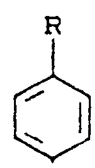

(I),

dans laquelle
R représente un groupe alkyle à chaîne droite ou ramifiée en $C_1$-$C_{12}$ ou un groupe cycloalkyle en $C_3$-$C_8$,
en présence de catalyseurs de Friedel-Crafts et en présence de catalyseurs auxiliaires, en phase liquide, caractérisé en ce que l'on utilise en tant que catalyseurs auxiliaires des imines cycliques benzocondensées de formule

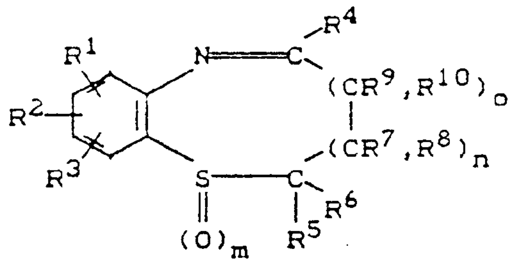

(II),

dans laquelle

$R^1$ et $R^2$ représentent chacun, indépenoamment l'un de l'autre, l'hydrogène, un groupe hydroxy, amino, cyano, un halogène, un groupe nitro, un groupe carboxyle, halogénocarbonyle, carboxamide, alcoxycarbonyle, alkyle, aryle, alcoxy, aryloxy, acyloxy, alkylthio, arylthio, acylthio, acyle, thioacyle ou acylamino,

$R^3$ représente l'hydrogène ou le chlore et peut en outre former avec l'un des groupes $R^1$ or $R^2$ dans le cas d'une substitution voisine et avec les atomes de carbone substitués un noyau condensé saturé, insaturé ou aromatique isocyclique ou hétéroryclique,

$R^4$ représente l'hydrogéne, un groupe alkyle, aryle, un halogène, un groupe alkylthio, arylthio, alcoxy, aryloxy, amino, hydrazino, alkylhydrazino ou phénylhydrazino,

m, n et o sont égaux chacun, indépendamment les uns des autres, à 0 ou 1,

$R^5$, $R^7$ et $R^9$ représentent chacun, indépendamment les uns des autres, l'hydrogène, un groupe alkyle, alcoxy, phényle, acyloxy, cyano, un halogène, un groupe carboxyle, alcoxycarbonyle, phénoxy ou acyle et

$R^6$, $R^8$ et $R^{10}$ représentent chacun, indépendamment les uns des autres, l'hydrogène, un groupe alkyle ou un halogène, ou bien $R^5$ et $R^7$ ou $R^7$ et $R^9$ peuvent représenter ensemble et avec les atomes de carbone substitués un noyau saturé, insaturé ou aromatique isocyclique ou hétérocyclique,

$R^6$ et $R^8$ ou $R^8$ et $R^{10}$ pouvant également représenter ensemble une double liaison, et $R^5$ et $R^6$ pouvant également représenter ensemble l'oxygène, le soufre ou l'azote à substituant $R^{11}$, reliés par une double liaison, $R^{11}$ représertant un groupe alkyle, aryle, acyle, alkylamino ou arylamino,

ou des benzo[f]-1,4-thiazépines de formule

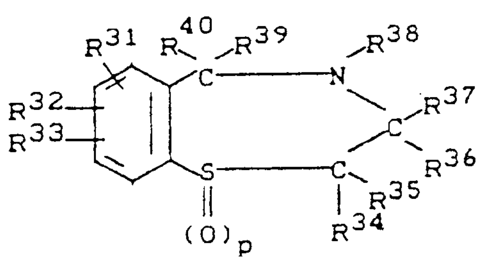

(III),

dans laquelle

$R^{31}$ et $R^{32}$ représentent chacun, indépendamment l'un de l'autre, l'hydrogène, un groupe hydroxy, amino, cyano, un halogène, un groupe nitro, alkyle en $C_1$-$C_8$, un groupe phényle non substitué ou substitué par $R^{31}$ et $R^{32}$ (à l'exception d'une nouvelle substitution par un groupe phényle substitué par $R^{31}$ et $R^{32}$), un groupe alcoxy en $C_1$-$C_8$, phénoxy, acyloxy en $C_1$-$C_8$, acyle en $C_1$-$C_8$ ou (alcoxy en $C_1$-$C_8$)-carbonyle,

$R^{33}$ représente l'hydrogène ou le chlore et peut en outre former avec l'un des groupes $R^{31}$ ou $R^{32}$ et avec les atomes de carbone substitués un noyau condensé saturé, insaturé ou aromatique isocyclique ou hétérocyclique de 5 à 8 chaînons,

$R^{34}$, $R^{36}$ et $R^{40}$ représentent chacun, indépendamment les uns des autres, l'hydrogène, un groupe alkyle en $C_1$-$C_8$, un groupe phényle non substitué ou substitué par $R^{31}$ et $R^{32}$ (à l'exception d'une nouvelle substitution par un groupe phényle substitué par $R^{31}$ et $R^{32}$), un groupe acyle en $C_1$-$C_8$, (alcoxy en $C_1$-$C_8$)-carbonyle, cyano, un halogène, un groupe carboxyle, alcoxy en $C_1$-$C_8$, alkylthio en $C_1$-$C_8$, phénylthio, benzylthio, phénoxy ou acy-

loxy en $C_1$-$C_8$,
$R^{35}$, $R^{37}$ et $R^{39}$ représentent chacun, indépendamment les uns des autres, l'hydrogène, un groupe alkyle en $C_1$-$C_8$, un halogène, un groupe alcoxy en $C_1$-$C_8$ ou alkylthio en $C_1$-$C_8$,
$R^{38}$ représente l'hydrogène, un groupe alkyle en $C_1$-$C_8$, un groupe phényle non substitué ou substitué par $R^{31}$ et $R^{32}$ (à l'exception d'une nouvelle substitution par un groupe phényle substitué par $R^{31}$ et $R^{32}$), un groupe acyle en $C_1$-$C_8$, thioacyle en $C_1$-$C_8$, halogénocarbonyle ou (alcoxy en $C_1$-$C_8$)-carbonyle et
p est égal à 0 ou 1,
les paires de substituants $R^{34}$ et $R^{35}$, $R^{36}$ et $R^{37}$ et $R^{39}$ et $R^{40}$ pouvant également représenter, indépenoamment les unes des autres, l'oxygène, le soufre ou l'azote à substituant $R^{38}$, reliés par une double liaison et
les paires de substituants $R^{35}$ et $R^{36}$, d'une part, $R^{38}$ et $R^{39}$, d'autre part, pouvant représenter, indépendamment l'une de l'autre, une double liaison et
les paires de substituants $R^{34}$ et $R^{37}$, d'une part, $R^{38}$ et $R^{39}$, d'autre part, pouvant représenter, indépendamment l'une de l'autre, des groupes alkylène de 3 à 5 chaînons dans lesquels un ou deux atomes de carbone peuvent être remplacés par l'oxygène, le soufre ou l'azote à substituant $R^{38}$ et
$R^{40}$
peut également représenter un groupe hydrazino, (alkyle en $C_1$-$C_8$)-hydrazino ou phénylhydrazino.

2. Procédé selon la revendication 1, caractérisé en ce que l'on utilise des imines cycliques benzocondensées de formules

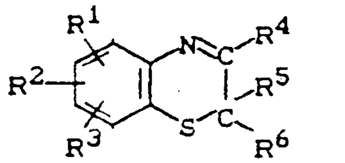

(IV),

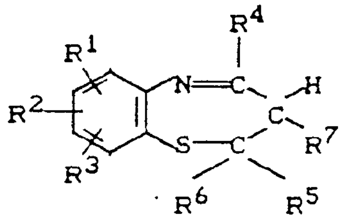

(V),

et/ou

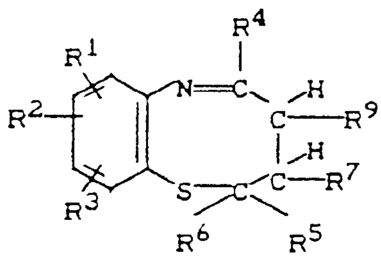

(VI),

dans lesquelles les symboles $R^1$ à $R^9$ ont les significations indiquées dans la revendication 1.

3. Procédé selon la revendication 2, caractérisé en ce que les symboles $R^1$, $R^2$ et $R^3$ sont remplacés par les symboles $R^{12}$, $R^{13}$ et $R^{14}$ parmi lesquels
$R^{12}$ et $R^{13}$ représentent chacun, indépendamment l'un de l'autre, l'hydrogène, un halogène, un groupe nitro, alkyle, aryle, alcoxy, aryloxy, acyloxy, alkylthio, arylthio, acyle ou thioacyle et
$R^{14}$ représente l'hydrogène ou le chlore et peut en outre former avec l'un des groupes $R^{12}$ et $R^{13}$ et avec les atomes de carbone portant ces substituants un noyau condensé saturé isocyclique de 5 à 7 chaînons ou un noyau benzénique condensé,
et de préférence les symboles $R^{12}$, $R^{13}$ et $R^{14}$ sont remplacés par les symboles $R^{21}$, $R^{22}$ et $R^{23}$ pour lesquels $R^{21}$ et $R^{22}$ représentent chacun, indépendamment l'un de l'autre, l'hydrogène, un groupe alkyle en $C_1$-$C_4$, alcoxy en $C_1$-$C_4$, le fluor ou le chlore et

$R^{23}$ représente l'hydrogène ou le chlore et peut en outre former avec l'un des groupes $R^{21}$ ou $R^{22}$ et avec les atomes de carbone portant ces substituants un noyau condensé cyclopentane, cyclohexane ou benzène.

4. Procédé selon la revendication 2, caractérisé en ce que le symbole $R^4$ est remplacé par le symbole $R^{15}$ qui représente l'hydrogène, un groupe alkyle en $C_1$-$C_4$, alcoxy en $C_1$-$C_4$, phényle, benzyle, le chlore, un groupe acyle en $C_1$-$C_4$, alkylthio en $C_1$-$C_4$, benzyloxy, benzylthio, hydrazino, (alkyle en $C_1$-$C_4$)-hydrazino ou phénylhydrazino,
et de préférence le symbole $R^{15}$ est remplacé par le symbole $R^{24}$ qui représente l'hydrogène, un groupe alkyle en $C_1$-$C_4$, benzyle, phényle, alcoxy en $C_1$-$C_4$, alkylthio en $C_1$-$C_4$, benzyloxy, benzylthio, hydrazino ou phénylhydrazino.

5. Procédé selon la revendication 2, caractérisé en ce que les symboles $R^5$, $R^6$, $R^7$ et $R^9$ sont remplacés par les symboles $R^{16}$, $R^{17}$, $R^{18}$ et $R^{19}$ qui représentent chacun, indépendamment les uns des autres, l'hydrogène, un groupe alkyle en $C_1$-$C_4$, le fluor ou le chlore, $R^{16}$, $R^{18}$ et $R^{19}$ pouvant en outre représenter des groupes alcoxy en $C_1$-$C_4$, acyle en $C_1$-$C_4$ ou phényle et pouvant en outre former, dans le cas d'une substitution voisine et avec les atomes de carbone portant ces substituants, un noyau cyclopentane, cyclohexane ou benzène.

6. Procédé selon la revendication 2, caractérisé en ce que l'on utilise des imines cycliques benzocondensées de formule

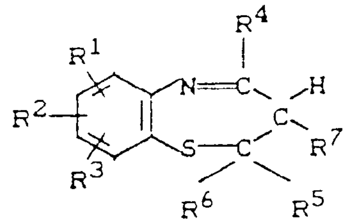

(V),

dans laquelle $R^1$ à $R^7$ ont les significations indiquées dans la revendication 2.

7. Procédé selon la revendication 1, caractérisé en ce que l'on utilise en tant que catalyseurs auxiliaires des benzo[f]-1,4-thiazépines de formule

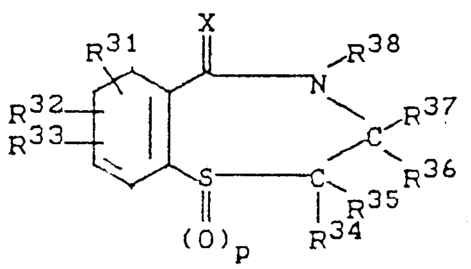

(VII),

dans laquelle
X représente l'oxygène, le soufre ou l'azote à substituant $R^{38}$, reliés par une double liaison et
$R^{31}$, $R^{32}$, $R^{33}$, $R^{34}$, $R^{35}$, $R^{36}$, $R^{37}$, $R^{38}$ et p ont les significations indiquées dans la revendication 1.

8. Procédé selon la revendication 1, caractérisé en ce que l'on utilise des catalyseurs auxiliaires de formule

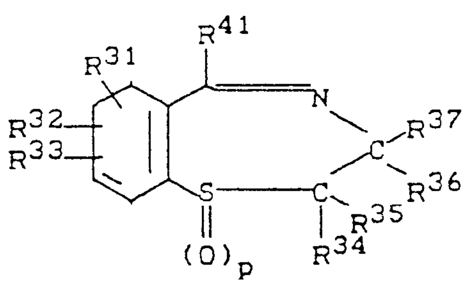

(VIII),

dans laquelle
$R^{41}$ représente un groupe alkyle en $C_1$-$C_8$, alcoxy en $C_1$-$C_8$, alkylthio en $C_1$-$C_8$, hydrazino, β-phénylhydrazino ou β-(alkyle en $C_1$-$C_8$)-hydrazino et

$R^{31}$, $R^{32}$, $R^{33}$, $R^{34}$, $R^{35}$, $R^{36}$, $R^{37}$ et p ont les significations indiquées dans la revendication 1.

9. Procédé selon la revendication 1, caractérisé en ce que l'on utilise des catalyseurs auxiliaires de formule

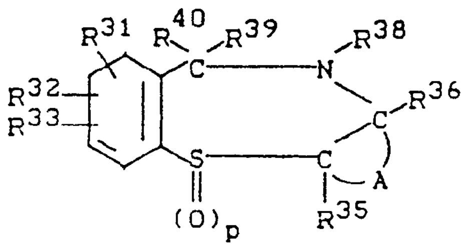

(IX),

dans laquelle
A représente un groupe triméthylène, tétraméthylène ou pentaméthylène et
$R^{31}$, $R^{32}$, $R^{33}$, $R^{35}$, $R^{36}$, $R^{38}$, $R^{39}$, $R^{40}$ et p ont les significations indiquées dans la revendication 1.

10. Procédé selon la revendication 1, caractérisé en ce que p est égal à 0.

11. Procédé selon la revendication 1, caractérisé en ce que les symboles $R^{31}$, $R^{32}$ et $R^{33}$ sont remplacés par les symboles $R^{42}$, $R^{43}$ et $R^{44}$ pour lesquels
$R^{42}$ et $R^{43}$ représentent chacun, indépendamment l'un de l'autre, l'hydrogène, un halogène, un groupe nitro, alkyle, aryle, alcoxy, aryloxy, acyloxy, alkylthio, arylthio, acyle ou thioacyle et
$R^{44}$ représente l'hydrogène ou le chlore et peut en outre former avec l'un des groupes $R^{42}$ et $R^{43}$ et avec les atomes de carbone portant ces substituants un noyau condensé saturé isocyclique de 5 à 7 chaînons ou un noyau benzénique condensé,
et de préférence caractérisé en ce que les symboles $R^{42}$, $R^{43}$ et $R^{44}$ sont remplacés par les symboles $R^{51}$, $R^{52}$ et $R^{53}$ respectivement pour lesquels
$R^{51}$ et $R^{52}$ représentent chacun, indépendamment l'un de l'autre, l'hydrogène, un groupe alkyle en $C_1$-$C_4$, alcoxy en $C_1$-$C_4$, le fluor ou le chlore et
$R^{53}$ représente l'hydrogène ou le chlore, et peut en outre former avec l'un des groupes $R^{51}$ ou $R^{52}$ et avec les atomes de carbone portant ces substituants un noyau condensé cyclopentane, cyclohexane ou benzène.

12. Procédé selon la revendication 1, caractérisé en ce que la quantité de catalyseur auxiliaire mise en oeuvre est de 0,0001 à 0,5% en poids, de préférence de 0,0005 à 0,1% en poids et tout spécialement de 0,0005 à 0,0075% en poids, par rapport à l'hydrocarbure aromatique mis en oeuvre.